(19) Europäisches Patentamt
European Patent Office
Office européen des brevets

(11) Veröffentlichungsnummer: **0 055 458**
**B1**

(12) **EUROPÄISCHE PATENTSCHRIFT**

(45) Veröffentlichungstag der Patentschrift: **13.02.85**

(51) Int. Cl.[4]: **C 07 D 277/42,** C 07 D 277/18, A 61 K 31/425 // C07C157/09

(21) Anmeldenummer: **81110677.2**

(22) Anmeldetag: **22.12.81**

(54) **Thiazolinderivate, Verfahren zu ihrer Herstellung, ihre Verwendung sowie pharmazeutische Präparate auf Basis dieser Verbindungen.**

(30) Priorität: **30.12.80 DE 3049460**

(43) Veröffentlichungstag der Anmeldung: **07.07.82 Patentblatt 82/27**

(45) Bekanntmachung des Hinweises auf die Patenterteilung: **13.02.85 Patentblatt 85/7**

(84) Benannte Vertragsstaaten: **AT BE CH DE FR GB IT LI LU NL SE**

(56) Entgegenhaltungen:
**EP - A - 0 023 964**
**FR - A - 2 319 345**
**FR - A - 2 327 778**

(73) Patentinhaber: **HOECHST AKTIENGESELLSCHAFT, Postfach 80 03 20, D-6230 Frankfurt am Main 80 (DE)**

(72) Erfinder: **Lang, Hans-Jochen, Dr., Rüdesheimer Strasse 7, D-6238 Hofheim am Taunus (DE)**
Erfinder: **Seuring, Bernhard, Dr., Johannesallee 20, D-6230 Frankfurt am Main 80 (DE)**
Erfinder: **Granzer, Ernold, Dr. Dr., Falkensteiner Strasse 24, D-6233 Kelkheim (Taunus) (DE)**

**EP 0 055 458 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

**Beschreibung**

Die Erfindung betrifft Verbindungen der allgemeinen Formel I

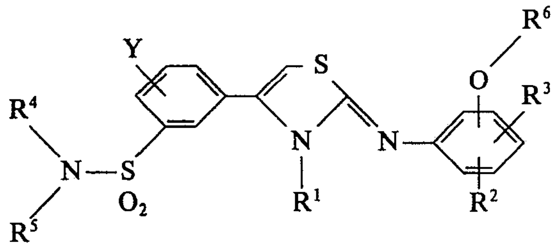

(I)

die als solche oder in Form ihrer pharmakologisch verträglichen Salze wertvolle pharmakologische Eigenschaften besitzen und daher als Arzneimittel geeignet sind. In der Formel bedeuten:

$R^1$ Alkyl mit 1 bis 3 C-Atomen,
$R^2$ und $R^3$ Wasserstoff, Halogen, Alkyl oder Alkoxy mit jeweils 1 bis 4 C-Atomen, wobei $R^2$ und $R^3$ gleich oder verschieden sind,
$R^4$ und $R^5$ Wasserstoff oder Alkyl mit 1 bis 4 C-Atomen, wobei $R^4$ und $R^5$ gleich oder verschieden sind, oder zusammen mit dem N-Atom einen gesättigten Ring mit bis zu 6 Ringgliedern bilden,
$R^6$ Wasserstoff oder Acyl mit 1 bis 4 C-Atomen,
Y Wasserstoff, Halogen oder Methyl.

Die Erfindung betrifft weiterhin ein Verfahren zur Herstellung der Verbindungen der allgemeinen Formel I, dadurch gekennzeichnet, daß man

a) Verbindungen der allgemeinen Formel II

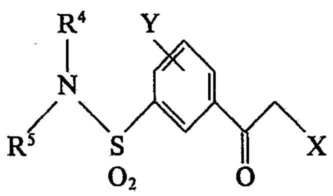

(II)

worin $R^4$, $R^5$ und Y die angegebene Bedeutung besitzen, X eine leaving group wie Halogen,

$CH_3SO_2$—O— oder 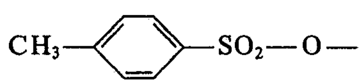

ist unter kondensierenden Reaktionsbedingungen mit einem Thioharnstoff der allgemeinen Formel III

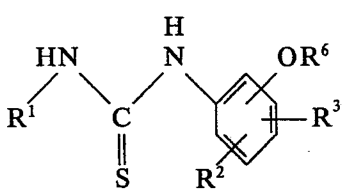

(III)

worin $R^1$, $R^2$, $R^3$, $R^6$ die angegebene Bedeutung besitzen, umsetzt, oder

b) aus Verbindungen der allgemeinen Formel IV

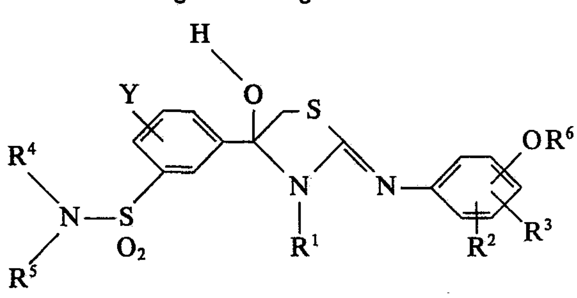

(IV)

worin $R^1$ bis $R^6$ und Y die angegebene Bedeutung besitzen, Wasser abspaltet oder

c) Verbindungen der allgemeinen Formel V

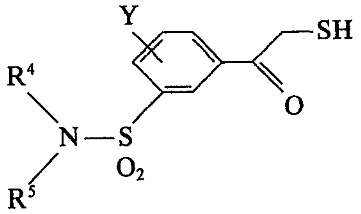

(V)

mit Verbindungen der allgemeinen Formel VI

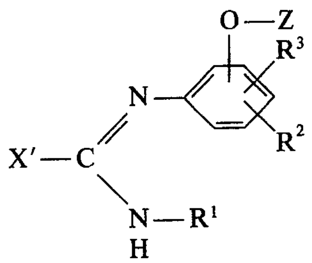

(VI)

zur Reaktion bringt, wobei $R^1$ bis $R^5$ die angegebene Bedeutung haben, Z eine Schutzgruppe der phenolischen Hydroxylgruppe, wie z. B. eine Acylgruppe mit 1 bis 4 C-Atomen, Methyl oder tert. Butyl bedeutet und X' eine leaving group, wie beispielsweise Halogen, Methoxy- oder Methylthio ist, oder

d) Verbindungen der allgemeinen Formel VII

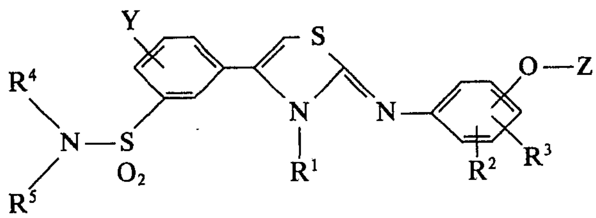

(VII)

worin $R^1$ bis $R^5$ und Y die zu Formel I und Z die zu Formel VI angegebene Bedeutung besitzen, durch eine hydrolytische oder solvolytische Spaltung in die Verbindungen der Formel I überführt, worin $R^6$ die Bedeutung von Wasserstoff hat, oder

e) Verbindungen der allgemeinen Formel I, worin $R^1$ bis $R^5$ und die angegebene Bedeutung besitzen und $R^6$ für Wasserstoff steht, mit einem Acylierungsmittel wie einem Acylchlorid oder Acylanhydrid in Verbindungen der Formel I mit $R^6$ in der Bedeutung von Acyl mit 1 bis 4 C-Atomen überführt, und gegebenenfalls eine der nach Weg a) bis e) erhaltenen Verbindungen der allgemeinen Formel I mit organischen oder anorganischen Säuren der allgemeinen Formel H—A in ihre Säureadditionssalze oder erhaltene Salze der Verbindungen der allgemeinen Formel I mit Basen in die freien basischen Verbindungen der Formel I überführt, oder eine erhaltene Verbindung der Formel I, worin $R^6$ Wasserstoff bedeutet, mit organischen oder anorganischen Basen in Phenolatsalze oder entsprechend erhaltene Salze mit Säuren in die freien basischen Verbindungen der Formel I überführt.

Als anorganische Säuren H—A kommen beispielsweise in Betracht: Halogenwasserstoffsäuren wie Chlorwasserstoffsäure und Bromwasserstoffsäure, sowie Schwefelsäure, Phosphorsäure und Amidosulfonsäure.

Als organische Säuren H—A seien beispielsweise Methansulfonsäure und p-Toluolsulfonsäure genannt.

Als anorganische Basen für die Phenolatsalzbildung kommen beispielsweise Natronlauge (NaOH) oder Kalilauge (KOH), als organische Basen kommen beispielsweise Natriummethylat, Natriumäthylat, Kalium-tert.butylat oder Tetraäthylammoniumhydroxid in Betracht.

Die Verbindungen der Formel IV sind neu. Die Erfindung betrifft daher weiterhin Verbindungen der Formel IV

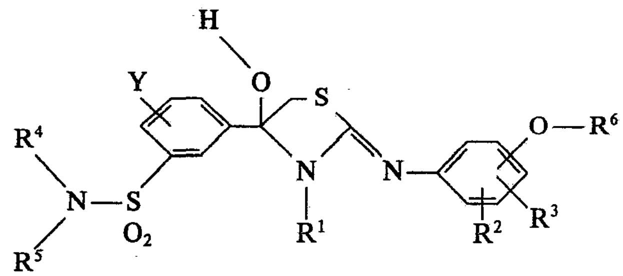

(IV)

worin $R^1$ bis $R^6$ und Y die zu Formel I angegebenen Bedeutungen haben, bzw. deren Säureadditionssalze und Phenolatsalze. Sie sind als Vorprodukte bei der Herstellung von Verbindungen der allgemeinen Formel I geeignet.

Die erfindungsgemäßen Verbindungen der Formel I können außerdem in ihren möglichen isomeren Strukturen vorliegen, wobei zum Zwecke der Vereinfachung nur eine der möglichen isomeren Formen einer jeweiligen Substanz angegeben wird.

Die unter a) bezeichnete Verfahrensweise wird vorteilhaft so ausgeführt, daß man die Verbindungen II mit den Thioharnstoffen III im molaren Verhältnis 1 : 1 bis 1 : 1,5 umsetzt. Mit größeren molaren Überschüssen an Thioharnstoff werden im allgemeinen keine nennenswerten Vorteile erzielt.

Die Reaktion wird vorteilhaft in inerten polaren organischen Lösungsmitteln wie Dimethylformamid, Dimethylacetamid, Äthylenglykoläthern, Aceton oder Tetrahydrofuran, besonders vorteilhaft in stark polaren protischen Lösungsmitteln wie Methanol, Äthanol, Isopropanol, n-Butanol, Essigsäure, Propionsäure, Ameisensäure sowie in Gemischen der genannten Solventien mit Wasser durchgeführt, wie sich auch wasserfreie Gemische der genannten Solventien eignen. Ebenso kann man die Reaktion auch ohne Verwendung eines Lösungsmittels durch Erwärmen des Reaktionsgemisches auf einen Temperaturbereich zwischen 80 und 220°C, bevorzugt zwischen 100 und 180°C durchführen. Bei Verwendung eines Lösungsmittels arbeitet man in einem bevorzugten Temperaturbereich von 50 bis 150°C.

Die Reaktionsdauer ist weitgehend vom Lösungsmittel und der angewendeten Reaktionstemperatur abhängig und liegt im allgemeinen zwischen 15 Minuten und 24 Stunden. Der quantitative Reaktionsablauf zu den erfindungsgemäßen Verbindungen I wird vorteilhaft dünnschichtchromatographisch an Kieselgelplatten verfolgt.

Vielfach scheiden sich die erfindungsgemäßen Verbindungen I filtrierbar in Form ihrer Säureadditionssalze im Verlauf der Reaktion schwerlöslich ab, andernfalls wird das Solvens verdampft, wobei gegebenenfalls durch nachträglichen Zusatz eines geeigneten Fällungsmittels, wie beispielsweise Essigester, Diäthyläther, Diisopropyläther, Aceton, Acetonitril die Ausbeute erhöht werden kann.

Die verwendeten Thioharnstoffe III werden in bekannter Weise durch Umsetzung von Aminen mit Isothiocyanaten, Schwefelkohlenstoff oder Thiophosgen dargestellt (vergl. Houben-Weyl, »Methoden der organischen Chemie«, Bd. 9, S. 384, 4. Auflage, Georg-Thieme-Verlag, Stuttgart, 1955).

Die Verbindungen der allgemeinen Formel II können nach mehreren in der Literatur beschriebenen Methoden gewonnen werden (vergl. z. B. DE-OS 2 436 263).

Nach der unter b) aufgeführten Verfahrensweise werden 2-Arylimino-4-hydroxy-4-(3-sulfamoylphenyl)-thiazolidine (IV) thermisch, bevorzugt durch Protonenkatalyse, zu den erfindungsgemäßen Verbindungen der allgemeinen Formel I dehydratisiert. Man arbeitet dabei vorteilhaft in polaren organischen Lösungsmitteln, wobei sich protische Lösungsmittel wie beispielsweise Methanol, Äthanol, Propanol, iso-Propanol, 1- bzw. 2-Butanol, Äthylenglykolmonomethyläther, Diäthylenglykolmonomethyläther oder niedere aliphatische Carbonsäuren wie Essigsäure, Propionsäure, Ameisensäure oder auch Gemische der genannten Solventien untereinander oder mit Wasser eignen.

Als Katalysator können anorganische oder organische Protonensäuren, insbesondere eine der als Solvens genannten aliphatischen Carbonsäuren, verwendet werden. Die Dehydratisierung der Verbindungen IV kann grundsätzlich auch ohne Anwendung eines Katalysators wie auch ohne Anwendung eines Lösungsmittels durchgeführt werden.

Man arbeitet in einem Temperaturbereich zwischen 0 und 200°C, wobei tiefere Temperaturen zu langen Reaktionszeiten führen und bei höheren Temperaturen zunehmend die Gefahr des Auftretens von Nebenprodukten besteht. Bevorzugt arbeitet man zwischen 50 und 150°C, wobei besonders vorteilhaft die Reaktion in siedendem Methanol, Äthanol, Propanol, Aceton oder Eisessig durchgeführt wird.

Das Reaktionsgemisch arbeitet man vorteilhaft analog der in Verfahrensweise a) angegebenen Weise auf.

Die Verbindungen der allgemeinen Formel IV erhält man nach an sich bekannten Methoden, beispielsweise analog der in der DE-OS 2 436 263 angegebenen Verfahrensweisen. Dabei sollten möglichst milde Reaktionsbedingungen und Reaktionstemperaturen sowie die Aufarbeitungsbedingungen unterhalb 40°C gewählt werden, wenn man die Herstellung möglichst reiner Verbindungen der Formel IV anstrebt.

Nach Verfahrensweise c) bringt man Verbindungen der allgemeinen Formel V mit Verbindungen der Formel VI vorteilhaft in einem polaren organischen Lösungsmittel, wie beispielsweise in niederen Alkoholen mit 1 bis 4 C-Atomen, Äthylenglykolmono- und Äthylenglykoldimethyläther, Diäthylenglykol-

mono- oder Diäthylenglykoldimethyläther, Aceton, Tetrahydrofuran, Essigester, Dimethylformamid, zur Reaktion.

Man führt die Umsetzung vorteilhaft zwischen 0 und 80°C, vorzugsweise zwischen 15 und 40°C durch und erwärmt nach Abklingen der exothermen Reaktion bis zur vollständigen Bildung der Verbindungen der Formel I auf Temperaturen zwischen 60° und 140°C. Der Reaktionsablauf wird zweckmäßigerweise dünnschichtchromatographisch an Kieselgelplatten verfolgt. Die Reaktionsdauer liegt zwischen 5 und 60 Stunden. Als besonders geeignet für diese Reaktion erweisen sich insbesondere Verbindungen V, in denen $R^4$ und $R^5$ organische Reste der angegebenen Bedeutung darstellen, sowie Verbindungen VI.

Die in Verfahrensweise c) verwendeten Verbindungen der Formel V können nach literaturbekannten Methoden hergestellt werden (z. B. DE-OS 2 436 263). Ebenso ist die Darstellung von Verbindungen der allgemeinen Formeln VI in der Literatur beschrieben (z. B. Chem. Ber. 97, 1232 (1964).

Zur Durchführung von Verfahrensweise d) unterwirft man Verbindungen der allgemeinen Formel VII, worin Z einen Acylrest bedeutet, in an sich bekannter Weise einer Solvolyse, indem man die Verbindungen VII bei Temperaturen zwischen 20 und 100°C, bevorzugt zwischen 30 und 60°C, mit der Lösung eines anorganischen Hydroxids M—OH mit M in der Bedeutung von z. B. Li, Na, K oder mit Ammoniak oder der Lösung eines organischen primären oder sekundären Amins wie Methylamin, Dimethylamin, Äthylendiamin oder Morpholin in Wasser.

Methanol, Äthanol, Isopropanol, in Dioxan, Tetrahydrofuran, Chloroform, Äthylglykolmono- und -dimethyläther oder in Mischungen der angegebenen Lösungsmittel behandelt, wobei das Arbeiten unter einem Inertgas, wie Stickstoff oder Argon empfohlen werden kann. Bei der Aufarbeitung des Reaktionsgemisches verfährt man vorzugsweise so, daß man Lösungsmittel im Vakuum unter vermindertem Druck vertreibt, den Rückstand in Wasser aufnimmt, mit einer Säure wie Essigsäure oder Salzsäure in der Wärme zwischen 40 und 80°C auf pH 4—6 stellt und den Niederschlag abfiltriert.

Hat Z die Bedeutung eines Alkyrestes, so spaltet man die Ätherderivate der allgemeinen Formel VII vorzugsweise im sauren Medium.

Besonders günstig erweist sich die Verwendung der tert.Butyläther (mit Z = tert.Butyl), die rasch in anorganischen oder organischen Säuren, z. B. in Trifluoressigsäure oder in Bortrifluorid-haltigem Eisessig oder in methanolischer Salzsäure bei Temperaturen zwischen 20 und 80°C gespalten werden. Der Fortgang der Reaktion wird vorteilhaft dünnschichtchromatographisch verfolgt. Zur Aufarbeitung des Reaktionsgemisches vertreibt man das Lösungsmittel unter vermindertem Druck, bringt den Rückstand nach Zugabe von Wasser vorzugsweise mit einer schwachen Base wie beispielsweise mit Ammoniumacetat auf pH 5 bis 6 und filtriert den Niederschlag ab.

Es ist zweckmäßig darauf zu achten, daß bei den alkalischen Spaltungen des Restes —OZ ein Mol Base benötigt wird und im Fall, daß $R^4$ und/oder $R^5$ Wasserstoff bedeuten, vorteilhaft mit je einem oder zwei weiteren Mol basischen Reagens gearbeitet wird.

Bei der Durchführung von Verfahrensweise e) bringt man Verbindungen der Formel I, worin $R^6$ Wasserstoff bedeutet, mit einem Acylierungsmittel wie Keten, vorzugsweise aber mit Acylanhydrid oder Acylchlorid in an sich bekannter Weise zur Reaktion, wobei sich die Bedingungen der Schotten-Baumann Reaktion (Ullmanns Encyklopädie der technischen Chemie, 3. Band, S. 88, Verlag Urban und Schwarzenberg, München-Berlin (1953), Houben-Weyl, Methoden der Organischen Chemie, Georg-Thieme Verlag Stuttgart, 1952, Band 8, Seite 545, 655; N. O. V. Sonntag, Chem. Rev. 52, 272 [1953]) besonders bewährt haben. Als bevorzugte Hilfsbasen verwendet man Pyridin, Triäthylamin oder Natronlauge, wobei man zweckmäßig innerhalb eines Temperaturbereiches von 20 bis 140°C arbeitet.

Die Verbindungen der Formel I können in einem geeigneten Lösungsmittel mit einer Säure der Formel H—A reversibel umgesetzt werden. Man kann dabei die Verbindungen I in die reinen Säuren, bevorzugt bei Temperaturen zwischen 0° und 60°C eintragen, sofern diese flüssig sind bzw. einen nicht wesentlich höheren Schmelzpunkt als 60°C besitzen und sofern sie keine Nebenreaktionen veranlassen. Vorteilhaft arbeitet man aber in einem Lösungsmittel, wie beispielsweise in Wasser, Dioxan, Tetrahydrofuran, Äther, einem Essigsäure-niederalkylester mit 1 bis 4 C-Atomen im Alkylteil, Acetonitril, Aceton, Methyläthylketon usw., wobei sich niedere Alkohole mit 1 bis 4 und Carbonsäuren mit 2 bis 4 C-Atomen als besonders geeignet erwiesen. Dabei werden pro Mol der Verbindungen I 1—1,5 Mol der Säuren H—A angewendet, man kann aber auch größere Mengen an Säure verwenden. Zweckmäßigerweise arbeitet man bei Temperaturen zwischen 0° und 120°C, bevorzugt zwischen 10° und 60°C.

Zweckmäßigerweise isoliert man nach Arbeiten im wäßrigen Medium die erfindungsgemäßen Salze beim Erhalten einer Lösung durch schonendes Verdampfen des Wassers, vorzugsweise durch Gefriertrocknung. Beim Arbeiten in organischen Lösungsmitteln scheiden sich die Säureadditionssalze vielfach nach Zugabe der jeweiligen Säure H—A schwerlöslich ab. Wird eine Lösung erhalten, so bringt man die Säureadditionsverbindungen gegebenenfalls nach vorangehender Konzentrierung mit einem geeigneten Fällungsmittel zur Abscheidung. Als Fällungsmittel eignen sich die zum gleichen Zweck in Verfahren a) beschriebenen Solvenzien.

Die Säureadditionsprodukte fallen auch bei sehr hohem Reinigungsgrad sehr oft in Form zäher Öle oder amorpher glasartiger Produkte an. Diese amorphen Produkte lassen sich vielfach gegebenenfalls durch Erwärmen auf 40° bis 80°C unter Behandlung mit einem organischen Lösungsmittel zur Kristallisation bringen.

Die Säureadditionsprodukte können in einem geeigneten Lösungsmittel durch Behandlung mit Basen, insbesondere mit Triethylamin oder Natriumhydrogencarbonatlösung, zu den Verbindungen der allgemeinen Formel I deprotoniert werden. Beim Arbeiten im wäßrigen Medium scheiden sich die freien basischen Verbindungen I schwerlöslich ab und können durch Filtration oder Extraktion mit einem organischen Lösungsmittel, vorzugsweise mit Essigsäureäthylester, abgetrennt und isoliert werden. Als organische Reaktionsmedien eignen sich in besonderer Weise niedere Alkohole mit 1 bis 4 C-Atomen, vorzugsweise Methanol und Äthanol. Die Reaktion zu den Verbindungen I findet spontan statt. Die Reaktion wird zwischen −35° und 100°C, bevorzugt zwischen 0° und 60°C durchgeführt. Wird ein mit Wasser mischbares organisches Lösungsmittel verwendet, so fällt man gegebenenfalls nach vorangehender Konzentrierung des Reaktionsgemisches die freien Basen der Formel I durch Zugabe von Wasser aus. Bei Verwendung eines mit Wasser nicht mischbaren Lösungsmittels arbeitet man vorteilhafterweise so, daß man nach der Umsetzung das Reaktionsgemisch mit Wasser wäscht und das organische Lösungsmittel gegebenenfalls nach vorangehender Trocknung verdampft.

Läßt man auf Verbindungen der Formel I, worin mindestens einer der Substituenten $R^4$, $R^5$, $R^6$ Wasserstoff bedeutet, mindestens 1 Mol einer hinreichend starken Base einwirken, so erhält man unter Deprotonierung der Sulfonamidgruppe oder der phenolischen OH-Gruppe Salze der allgemeinen Formel I b

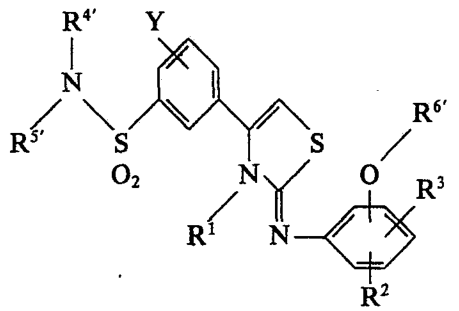

worin mindestens einer der Substituenten $R^{4'}$, $R^{5'}$ oder $R^{6'}$ das Kation eines Alkali- oder Erdalkalimetalls ist und die übrigen Substituenten $R^{4'}$, $R^{5'}$ bzw. $R^{6'}$ die zu $R^4$, $R^5$ bzw. $R^6$ angegebene Bedeutung haben.

Als Basen können Hydroxide der Alkali- und Erdalkalimetalle, vorzugsweise NaOH und KOH, Alkali- und Erdalkalialkoholate, $NaOCH_3$ und $NaOC_2H_5$, NaH, Natrium-methylsulfinylmethid usw. verwendet werden.

Als Lösungsmittel verwendet man Wasser oder polare organische Lösungsmittel wie Methanol, Äthanol, Isopropanol, n-Butanol, Dimethylformamid, Dimethylsulfoxid, Diäthylenglykol-dimethyläther, Acetonitril.

Durch Zugabe äquivalenter Mengen einer geeigneten Säure H—A erhält man die erfindungsgemäßen Verbindungen I zurück.

Diese reversible Säure-Base-Reaktion kann man zur Reinigung der Verbindungen I heranziehen.

Bevorzugt von den erfindungsgemäßen Verbindungen sind diejenigen der allgemeinen Formel I, in denen die Substituenten die nachfolgenden, in Tabelle 1 beschriebenen Bedeutungen besitzen:

Tabelle 1

| | | |
|---|---|---|
| $R^1$ | = | Methyl, Ethyl |
| $R^2$ | = | Wasserstoff, Chlor |
| $R^3$ | = | Wasserstoff, Chlor, Methyl |
| $R^4$ | = | Wasserstoff, Methyl, Ethyl |
| $R^5$ | = | Wasserstoff, Methyl |
| $R^6$ | = | Wasserstoff, $-COH_3$, $-COC_2H_5$ |
| Y | = | Brom, Chlor oder Methyl in 4-, 5- oder 6-Stellung zum Thiazolin-Ring, wobei der Sulfamoylrest in 3-Stellung festgelegt ist, |

wobei als besonders bevorzugte Verbindungen solche Verbindungen der Formel I in Betracht kommen, in denen die Substituenten die in Tabelle 2 angegebenen Bedeutungen besitzen:

Tabelle 2

| | | |
|---|---|---|
| $R^1$ | = | Methyl |
| $R^2$, $R^3$ | = | Wasserstoff |
| $R^4$, $R^5$ | = | Wasserstoff und/oder Methyl |

Fortsetzung

$R^6$ = Wasserstoff, wobei die OH-Gruppe in 4-Stellung zur Iminogruppe steht
Y = Chlor in 4-Stellung zum Thiazolinring

Erfindungsgemäß können außer den in den Ausführungsbeispielen und in den Tabellen 4 und 5 beschriebenen Thiazolinderivaten auch die in der folgenden Tabelle 3 zusammengestellten Verbindungen der allgemeinen Formel I a und IV a bzw. deren Säureadditionsprodukte erhalten werden:

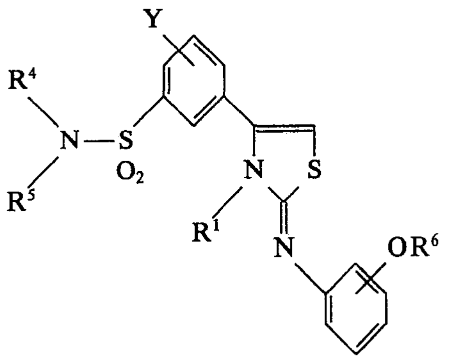

(Ia)

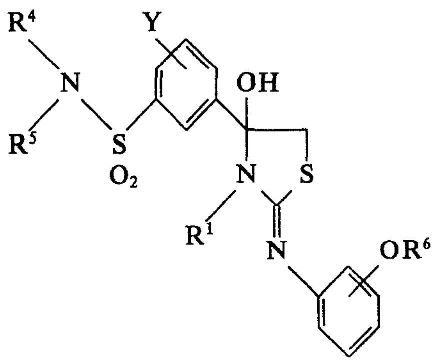

(IVa)

Tabelle 3

Zeichenerklärung:
Me = Methyl, Et = Ethyl, n-Pr = n-Propyl, i-Pr = Isopropyl, Bu = Butyl, t-Bu = tert.-Butyl, Ac = Acetyl, Prop = Propionyl, Ph = Phenyl, die vor dem Substituenten angegebenen Nummern bezeichnen seine Stellung am jeweiligen Phenylrest, wobei der Thiazolinring in 1- und der Sulfamoylrest in 3-Position festgelegt sind

| Lauf. Nr. | Y | $R^1$ | $R^4$ | $R^5$ | $OR^6$ |
|---|---|---|---|---|---|
| 1 | 5-Cl | Me | H | H | 4-OH |
| 2 | 5-Cl | Me | H | H | 3-OH |
| 3 | 5-Cl | Me | H | H | 2-OH |
| 4 | 5-Cl | Me | H | Me | 4-OH |
| 5 | 5-Cl | Me | H | Me | 3-OH |
| 6 | 5-Cl | Me | H | Me | 2-OH |
| 7 | 5-Cl | Me | Me | Me | 3-OH |
| 8 | 6-Cl | Me | H | H | 4-OH |

Fortsetzung

| Lauf. Nr. | Y | $R^1$ | $R^4$ | $R^5$ | $OR^6$ |
|---|---|---|---|---|---|
| 9 | 6-Cl | Me | H | H | 3-OH |
| 10 | 6-Cl | Me | H | H | 2-OH |
| 11 | 6-Cl | Me | H | Me | 4-OH |
| 12 | 6-Cl | Me | H | Me | 3-OH |
| 13 | 6-Cl | Me | H | Me | 2-OH |
| 14 | 6-Cl | Me | Me | Me | 3-OH |
| 15 | 6-Cl | Me | Me | Me | 4-OAc |
| 16 | 6-Cl | Me | Me | Me | 2-OAc |
| 17 | 6-Cl | Me | Me | Me | 3-OAc |
| 18 | 6-Cl | Me | Me | Me | 4-OProp |
| 19 | 5-Cl | Me | Me | Me | 4-OAc |
| 20 | 5-Cl | Me | Me | Me | 3-OAc |
| 21 | 4-Me | Me | H | H | 4-OH |
| 22 | 4-Me | Me | H | Me | 4-OH |
| 23 | 4-Me | Me | Me | Me | 3-OH |
| 24 | 4-Me | Me | Me | Me | 2-OH |
| 25 | 5-Me | Me | H | H | 4-OH |
| 26 | 5-Me | Me | Me | Me | 3-OH |
| 27 | 5-Me | Me | Me | Me | 2-OH |
| 28 | H | Me | H | H | 4-OH |
| 29 | H | Me | Me | Me | 4-OH |
| 30 | H | Me | H | Me | 4-OH |
| 31 | H | Me | H | H | 2-OH |
| 32 | H | Me | Me | Me | 3-OH |
| 33 | H | Me | Me | Me | 3-OH |
| 34 | H | Me | H | H | 3-OH |

Die erfindungsgemäßen Verbindungen der Formel I sind wertvolle Arzneimittel und zeichnen sich durch eine sehr günstige Wirkung auf die Serumlipoproteine aus. Sie können daher als Arzneimittel, insbesondere zur Beeinflussung der Serumlipoproteine verwendet werden. Die Erfindung betrifft daher auch pharmazeutische Präparate auf Basis der Verbindungen der Formel I und ihrer pharmakologisch verträglichen Salze sowie die Verwendung als Arzneimittel.

In der Literatur wird über eine anorektische, ZNS-stimulierende und diuretische Wirkung von 4-Phenyl-2,3-dihydrothiazolin-Derivaten berichtet, wobei es sich um Verbindungen ohne Sulfonamidsubstitution im Phenylteil handelt und die 2-Iminofunktion nicht durch Aryl substituiert ist (vergl. US-PS 3 671 533, DE-OS 1 938 674). Beschrieben sind auch 3-Alkyl-4-phenyl-2-phenylimino-4-thiazoline (vergl. Univ. Kansas Sci. Bull. 24, 45–49 [1936]), bei denen der in Position 4 befindliche Phenylrest keine Sulfonamidgruppe trägt. Unterschiedlich substituierte 4-(3-Sulfamoyl-phenyl)-3-alkyl-2-imino-4-thiazoline bzw. -thiazolidine werden ebenfalls in der Literatur erwähnt, und zwar insbesondere als Diuretika (vergl. »Diuretic Agents«, E. J. Cragoe, Jr., Editor; ACS-Symposium Series 83, Seite 24, Washington D. C., [1978]).

Es war nun überraschend, daß die erfindungsgemäßen Verbindungen der Formel I eine sehr starke und günstige Beeinflussung der Serumlipoproteine zeigen, während die in der oben genannten Literatur beschriebenen Thiazolinderivate keine oder nur geringe in qualitativer und quantitativer Hinsicht deutlich unterlegene Effekte verursachen.

Es ist allgemein anerkannt, daß für die Entstehung arteriosklerotischer Gefäßveränderungen, insbesondere der coronaren Herzkrankheit, Hyperlipoproteinämien einen wesentlichen Risikofaktor darstellen. Für die Prophylaxe und die Regression von atherosklerotischen Veränderungen kommt daher der Senkung erhöhter Serum-Lipoproteine eine außerordentliche Bedeutung zu. Hierbei kommt es aber auf ganz bestimmte Klassen von Serum-Lipoproteinen an, da die low density (LDL) und very low density-Lipoproteine (VLDL) einen atherogenen Risikofaktor darstellen, während die high density-Lipoproteine (HDL) eine Schutzfunktion gegenüber der coronaren Herzkrankheit darstellen. Hypolipidämika sollen demnach VLDL-Cholesterin und LDL-Cholesterin im Serum erniedrigen, dabei aber die HDL-Cholesterin-Konzentration nach Möglichkeit unbeeinflußt lassen oder sogar erhöhen. Die erfindungsgemäßen Verbindungen haben wertvolle therapeutischen Eigenschaften. So erniedrigen sie vor allem die Konzentration von LDL und VLDL, während die HDL-Fraktion entweder in wesentlich geringerem Maße erniedrigt, oder sogar erhöht wird. Sie können daher zur Prophylaxe und Regression von atherosklerotischen Veränderungen herangezogen werden, indem sie einen kausalen Risikofaktor ausschalten. Hierzu zählen nicht nur die primären Hyperlipoproteinämien, sondern auch gewisse sekundäre Hyperlipidämien wie sie z. B. beim Diabetes vorkommen. Das relative Lebergewicht wird durch die Verbindungen I nicht wesentlich verändert, während das als hypolipidämischer Standard verwendete Clofibrat zu einer starken Erhöhung des relativen Lebergewichts führt.

Die Wirkung z. B. der in der nachfolgenden Tabelle I angeführten Verbindungen auf die Serum-Lipoproteine wurde an männlichen Wistar-Ratten untersucht, die 7 Tage per Schlundsonde mit den erfindungsgemäßen in Polyäthylenglykol 400 suspendierten Verbindungen behandelt wurden. Außerdem wurde eine Kontrollgruppe, die nur das Lösungsmittel Polyäthylenglykol 400 erhielt, mitgeführt, sowie eine Ratten-Gruppe, die zum Vergleich das Standardhypolipidämikum Clofibrat in Polyäthylenglykol 400 gelöst, erhielt. Pro Gruppe wurden in der Regel 10 Tiere eingesetzt, denen am Ende der Behandlung das Blut nach leichter Äthernarkose aus dem Orbitalplexus entnommen wurde. Das daraus gewonnene Serum wurde gepoolt. Die Serum-Lipoproteine wurden in der Ultrazentrifuge in folgende Dichtklassen getrennt: VLDL 1,006; LDL 1,006 bis 1,04; HDL 1,04 bis 1,21.

Aus den in der Ultrazentrifuge isolierten Lipoprotein-Fraktionen wurde das darin enthaltene Cholesterin vollenzymatisch nach der CHOD-PAP-Methode mittels der Testkombination von Boehringer-Mannheim bestimmt und die Werte in μg/ml Serum umgerechnet. Die Veränderung des Lipoprotein-Cholesterins in der behandelten Gruppe gegenüber einer unter gleichen Bedingungen mitgeführten Kontrollgruppe wurde bestimmt. Es wurde beobachtet, daß Clofibrat eine etwa gleichstarke Senkung der LDL-Fraktion und HDL-Fraktion bewirkt, während die neuen Verbindungen eine starke selektiv senkende Wirkung auf die atherogenen Lipoproteinfraktionen (VLDL und LDL) ausüben und die schützende HDL-Fraktion im wesentlichen unbeeinflußt lassen oder sogar vermehren.

Tabelle I

Änderung der Serumlipoprotein-Spiegel bei Ratten nach 7tägiger p. o. Behandlung mit den Verbindungen

| Verbindung gemäß Beispiel | Dosis mg/kg/Tag | Veränderungen des Cholesterins (im Vergleich zur Kontrollgruppe) im Serum | in der Serumlipoprotein-Fraktion VLDL | LDL | HDL |
|---|---|---|---|---|---|
| 2 | 10 | − 9 | −54 | −17 | − 4 |
| 5 | 10 | + 2 | −24 | −20 | + 7 |
| 14 | 10 | −12 | −43 | −27 | − 8 |
| 8 | 30 | − 9 | −55 | −52 | +19 |
| 32 | 10 | −21 | −21 | −32 | − 6 |
| 42 | 10 | −25 | −68 | −44 | − 4 |

Als therapeutische Zubereitung der Verbindungen der Formel I kommen vor allem Tabletten, Dragees, Kapseln, Suppositorien und Säfte in Frage. Die neuen Verbindungen können dabei entweder allein oder mit pharmakologisch annehmbaren Trägern vermischt, angewandt werden. Eine orale Anwendungsform wird bevorzugt. Zu diesem Zweck werden die aktiven Verbindungen vorzugsweise mit an sich bekannten Substanzen vermischt und durch an sich bekannte Methoden in geeignete Darreichungsformen gebracht, wie Tabletten, Steckkapseln, wäßrige oder ölige Suspensionen oder wäßrige oder ölige Lösungen. Als inerte Träger können z. B. Magnesiumkarbonat, Milchzucker oder Maisstärke unter Zusatz anderer Stoffe wie z. B. Magnesiumstearat verwendet werden. Dabei kann die Zubereitung als Trocken- oder Feuchtgranulat erfolgen. Als ölige Trägerstoffe oder Lösungsmittel kommen besonders pflanzliche und tierische Öle in Betracht wie z. B. Sonnenblumenöl oder Lebertran. Als tägliche Dosis kommen etwa 50 mg bis 5 g in Betracht, vorzugsweise 100 bis 1000 mg. Eine Dosierungseinheit enthält vorzugsweise 50 bis 1000 mg, insbesondere 250 bis 500 mg.

Die Zubereitungen können bei der Behandlung von Lipidstoffwechselstörungen außer den üblichen Füll- und Trägerstoffen noch einen weiteren Wirkstoff wie z. B. ein Antihypertensivum, wie beispielsweise ein Saluretikum, Reserpin, Hydralazin, Guanethidin, α-Methyldopa, Clonidin oder ein β-Sympathikolytikum, oder ein antihyperurikämisch wirksames Mittel, ein orales Antidiabetikum, ein Geriatrikum oder eine Verbindung mit durchblutungssteigernder Wirkung enthalten.

Die reinen erfindungsgemäßen Vorprodukte der allgemeinen Formel IV zeigen im Vergleich zu den erfindungsgemäßen Verbindungen der Formel I — wenn überhaupt — deutlich schwächere Effekte auf die Serumlipoproteine, besitzen aber wie auch strukturverwandte Thiazolidinderivate (vergl. DE-OS 2 436 263) eine zum Teil sehr gute salidiuretische Wirksamkeit.

Die in den nachfolgenden Beispielen aufgeführten Schmelz- und Zersetzungspunkte sind nicht korrigiert.

## Beispiel 1

### 4-(4-Chlor-3-dimethylsulfamoylphenyl)-2-(4-hydroxy-phenylimino)-3-methyl-4-thiazolin-hydrobromid

a) 6,8 g (0,02 Mol) 2-Brom-4'-chlor-3'-dimethylsulfamoylacetophenon und 3,7 g (0,021 Mol) 1-(4-Hydroxyphenyl)-3-methyl-thioharnstoff werden in 100 ml Äthanol im Verlauf von 1 Stunde bis zum Sieden erhitzt. Nun versetzt man mit 50 ml Eisessig und erhitzt 2—3 weitere Stunden zum Sieden. Nach dem Abdestillieren des Lösungsmittels im Wasserstrahlvakuum versetzt man den Rückstand mit Diisopropyläther, Essigester oder Diäthyläther und filtriert ab. Farblose Kristalle, Schmp. 276—281°C (Zers.).

b) 5,23 g (0,01 Mol) 4-(4-Chlor-3-dimethylsulfamoylphenyl)-3-methyl-2-(4-hydroxyphenylimino)-thiazolidin-4-ol-hydrobromid werden in 70 ml Eisessig über eine Dauer von 20 Min. zum Sieden erhitzt. Nach dem Abkühlen vervollständigt man die Kristallisation durch Zugabe von ca. 150 ml Diisopropyläther, rührt eine weitere Stunde bei Raumtemperatur und filtriert ab. Farblose Kristalle, Schmp. 275—279°C (Zers.).

Beispiel 2

4-(4-Chlor-3-dimethylsulfamoylphenyl)-2-(4-hydroxyphenylimino)-3-methyl-4-thiazolin

a) 4,24 g (0,01 Mol) 4-(4-Chlor-3-dimethylsulfamoylphenyl)-2-(4-hydroxyphenylimino)-3-methyl-4-thiazolin-hydrobromid werden in 120 ml Methanol suspendiert und das Reaktionsgemisch nach Zugabe von 4 g (0,04 Mol) Triäthylamin 45 Min. bei Raumtemperatur gerührt. Nach Abdestillieren des Lösungsmittels unter vermindertem Druck bringt man durch Behandeln mit Wasser zur Kristallisation. Farblose bis schwach gelbe Kristalle, Schmp. 188—192°C (aus Äthanol).

b) 4,4 g (0,01 Mol) 4-(4-Chlor-3-dimethylsulfamoylphenyl)-2-(4-hydroxyphenylimino)-3-methylthiazolidin-4-ol werden in 60 ml Eisessig 1 Stunde zum Sieden erhitzt, das Lösungsmittel abdestilliert und der Rückstand unter Zusatz von Wasser zur Kristallisation gebracht. Schmp. 189—191°C.

c) Zu einer Mischung aus 2,9 g (10 mmol) 4′-Chlor-3′-dimethylsulfamoyl-acetophenon-2-thiol (vergl. DE-OS 2 436 263) und 2,2 g (10 mmol) N-Methyl-N′-(4-hydroxyphenyl)-chlorformamidin-hydrochlorid (Schmp. 205—206°C [Zers.]), hergestellt aus 1-Methyl-3-(4-hydroxyphenyl)-thioharnstoff und Phosgen in Tetrahydrofuran) in ca. 50 ml wasserfreiem Isopropanol tropft man im Verlauf von 30 min bei 10 bis 15°C unter Feuchtigkeitsausschluß eine Lösung von 2 g Triäthylamin in wenig Isopropanol. Nach Zugabe von ca. 50 ml Chloroform rührt man über Nacht bei Raumtemperatur, setzt 20 ml Eisessig zu und erhitzt 1 Std. unter Rückfluß. Nach Entfernen des Lösungsmittels im Vakuum nimmt man den Rückstand in ca. 50 ml Chloroform, wäscht mehrfach wenig Wasser und unterwirft die organische Phase nach Trocknen und Einengen der Säulenchromatographie (Kieselgel, Elutionsmittel Toluol/Essigester 1 : 10 bis 1 : 1). Aus den eingeengten Produktfraktionen erhält man ein beiges Pulver, nach Umkristallisation aus Essigester/Ethanol farblose Kristalle vom Schmp. 190—192°C.

d) Eine Suspension von 1,7 g 4-(4-Chlor-3-dimethylsulfamoylphenyl)-2-(4-acetoxyphenyl-imino)-3-methyl-4-thiazolin (Beispiel 8) in 30 ml Ethanol und 20 ml Wasser wird mit 2 N Natronlauge auf pH 11—12 gestellt und 3 h bei RT gerührt. Nach Neutralisation mit 2 N Salzsäure wird mit Essigsäuremethylester mehrfach ausgeschüttelt, die organischen Phasen getrocknet, eingeengt und der Rückstand aus Essigester/Ethanol umkristallisiert. Beigefarbene Kristalle vom Schmp. 188—190°C, nach Dünnschichtvergleich identisch mit dem nach Verfahrensweise a)—c) erhaltenen Produkt.

Beispiel 3

4-(4-Chlor-3-methylsulfamoylphenyl)-2-(4-hydroxyphenylimino)-3-methyl-4-thiazolin-hydrochlorid

a) Analog der in Beispiel 1 a) angegebenen Vorschrift durch Umsetzung von 2,4′-Dichlor-3′-methylsulfamoyl-acetophenon und 1-(4-Hydroxyphenyl)-3-methyl-thioharnstoff. Farblose Kristalle, Schmp. 302°C (Zers.).

b) 4,1 g 4-(4-Chlor-3-methylsulfamoylphenyl)-2-(4-hydroxyphenylimino)-3-methyl-4-thiazolin werden in 150 ml Methanol aufgeschlämmt und mit gesättigter ätherischer Chlorwasserstofflösung sauer gestellt, das Lösungsmittel abdestilliert und der Rückstand aus Äthanol umkristallisiert. Schmp. 300—302°C (Zers.).

Beispiel 4

4-(4-Chlor-3-dimethylsulfamoylphenyl)-2-(4-hydroxyphenylimino)-3-methyl-4-thiazolin-hydrochlorid

Erhält man analog der in Beispiel 3 a) und b) angegebenen Vorschrift. Farblose Kristalle, Schmp. 264°C (Zers.).

Beispiel 5

4-(4-Chlor-3-sulfamoylphenyl)-2-(4-hydroxyphenylimino)-3-methyl-4-thiazolin

Aus 6,2 g (20 mmol) 2-Brom-4′-chlor-3′-sulfamoylacetophenon und 3,7 g (21 mmol) 1-(4-Hydroxyphenyl)-3-methylthioharnstoff in 80 ml Aceton durch einstündiges Rühren bei Raumtemperatur und anschließendes Rühren unter Rückfluß während 1 Std. nach Zusatz von 100 ml Eisessig. Aus dem nach Einengen der Reaktionsmischung anfallenden Hydrobromid der Titelverbindung (Schmp. 274°C unter Zersetzung) wird durch Verrühren mit 100 ml gesättigter wäßriger Natriumbicarbonat-Lsg., Waschen mit Wasser und Umkristallisation aus Ethanol/Wasser das Produkt vom Schmp. 224—226°C erhalten.

## Beispiel 6

4-(3-Dimethylsulfamoyl-5-methylphenyl)-2-(4-hydroxyphenylimino)-3-methyl-4-thiazolin

5,9 g (12 mmol) 4-(3-Dimethylsulfamoyl-5-methylphenyl)-2-(4-hydroxyphenylimino)-3-methyl-4-thiazolin-hydrobromid werden in ca. 50 ml Methanol suspendiert und mit 5 ml Triethylamin versetzt. Aus der resultierenden roten Lösung fällt nach ca. 30 min Rühren bei Raumtemperatur ein heller Feststoff. Dieser wird nach kurzem Nachrühren abgesaugt und aus Isopropanol umkristallisiert. Farblose Kristalle, Schmp. 218—220°C.

Das Ausgangsmaterial ist in Beispiel 83 erwähnt und wurde analog Beispiel 1 a) hergestellt. Das hierfür benötigte 2-Brom-5′-methyl-3′-dimethylsulfamoylacetophenon ist in der DE-OS 2 926 771 beschrieben.

## Beispiel 7

4-(2-Chlor-5-dimethylsulfamoylphenyl)-2-(4-hydroxyphenylimino)-3-methyl-4-thiazolin

a) Erhält man analog der in Beispiel 2 a) angegebenen Vorschrift aus 4-(2-Chlor-5-dimethylsulfamoylphenyl)-2-(4-hydroxyphenylimino)-3-methyl-4-thiazolinhydrobromid (Beispiel 60) wobei man anstelle von Triethylamin mit einer kalten 20%igen methanolischen Ammoniaklösung alkalisch stellt, entsprechend Beispiel 2 a) aufarbeitet und das getrocknete kristalline Rohprodukt aus Ethanol umkristallisiert, Schmp. 220—222°C.

b) Erhält man analog der in Beispiel 2 c) angegebenen Vorschrift aus 2′-Chlor-5′-dimethyl-sulfamoylacetophenon-2-thiol (Schmp. 100—115°C) und N-Methyl-N′-(4-hydroxy-phenyl)-chlorformamidin-hydrochlorid. Schmp. 221—224°C (aus Ethanol).

## Beispiel 8

4-(4-Chlor-3-dimethylsulfamoylphenyl)-2-(4-acetoxyphenylimino)-3-methyl-4-thiazolin

2,1 g (5 mmol) 4-(4-Chlor-3-dimethylsulfamoylphenyl)-2-(4-hydroxyphenylimino)-3-methyl-4-thiazolin (Beispiel 2) werden in 25 ml Acetanhydrid suspendiert und die beim Erwärmen entstehende gelbe Lösung 1 Stunde bei 120°C gerührt. Nach Abdestillieren des Acetanhydrids im Vakuum wird der feste Rückstand aus Isopropanol umkristallisiert. Farblose Kristalle vom Schmp. 173—174°C.

## Beispiel 9

4-(4-Chlor-3-dimethylsulfamoylphenyl)-2-(4-propionyloxyphenylimino)-3-methyl-4-thiazolin

2 g (4,7 mmol) 4-(4-Chlor-3-dimethylsulfamoylphenyl)-2-(4-hydroxyphenylimino)-3-methyl-4-thiazolin werden in 40 ml trockenem Chloroform suspendiert. Man gibt nacheinander 0,4 g (4,7 mmol) Propionylchlorid (gelöst in 10 ml Chloroform) und 1,4 g (18 mmol) Pyridin zu. Unter schwach exothermer Reaktion erhält man kurzzeitig eine Lösung, aus der ein gelber Feststoff ausfällt. Nach 3stündigem Rühren bei Raumtemperatur gibt man weitere 0,4 ml Propionylchlorid zu. Die resultierende gelbe Lösung wird nach 3tägigem Stehenlassen bei Raumtemperatur im Vakuum zur Trockne eingeengt, der Rückstand zwischen Wasser und Essigester verteilt und der nach Einengen der getrokkneten organischen Phase verbleibende Rückstand aus Isopropanol umkristallisiert. Farblose Kristalle vom Schmp. 155—159°C.

## Beispiel 10

4-(3-Chlor-5-dimethylsulfamoylphenyl)-2-(4-hydroxyphenylimino)-3-methyl-4-thiazolin

Erhält man aus analog Bsp. 1 a) oder 1 b) hergestelltem 4-(3-Chlor-5-dimethylsulfamoylphenyl)-2-(4-hydroxyphenylimino)-3-methyl-4-thiazolin-Hydrobromid (Schmp. 285° [Zers.]) durch Umsetzung mit Triethylamin in Ethanol analog Bsp. 2 a), farbl. Kristalle, vom Schmp. 155—158°C.

Das für die Herstellung benötigte 2-Brom-3′-chlor-5′-dimethylsulfamoylacetophenon ist in der DE-OS 2 926 771 beschrieben.

Beispiel 11

4-(4-Chlor-3-dimethylsulfamoylphenyl)-2-(4-hydroxyphenylimino)-3-methyl-thiazolidin-4-ol-hydrobromid

6,8 g (0,02 Mol) 2-Brom-4'-chlor-3'-dimethylsulfamoylacetophenon werden in 60 ml Aceton oder 150 ml Ethanol oder 50 ml Essigester gelöst und nach Zugabe einer Lösung von 3,7 g (0,022 Mol) 1-(4-Hydroxyphenyl)-3-methyl-thioharnstoff in 60 ml Aceton oder 50 ml Ethanol oder 100 ml Essigester 1 bis 4 Stunden bei Raumtemperatur gerührt, die Kristalle abfiltriert und nach Waschen mit wenig Aceton und Ether im kalten Luftstrom oder im Vakuum über Paraffinstückchen getrocknet. Farblose Kristalle, Schmp. 255–264°C unter Zersetzung, bei langsamen Aufheizen; Schmp. 178–180°C, bei raschem Aufheizen.

Die in den Ausführungsbeispielen 1–10 beschriebenen Verfahrensweisen a) bis e) sind geeignet zur Herstellung der in Tabelle 4 beschriebenen Verbindungen (Ausführungsbeispiele 12 bis 104).

In zu Ausführungsbeispiel 11 analoger Weise erhält man die in Tabelle 5 beschriebenen Vor- bzw. Zwischenprodukte 105–149, deren Schmelzpunkte bzw. Zersetzungstemperaturen wegen der beim Aufheizen oft unmerklichen Wasserabspaltung und dem damit verbundenen möglichen Übergang in Verbindungen der allgemeinen Struktur I sehr von der Aufheizgeschwindigkeit beeinflußt sind.

Tabelle 4

(Erklärungen vergl. Tabelle 3)

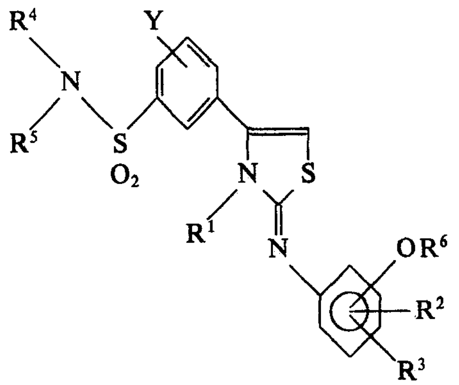

× HX

| Beisp.-Nr. | Y | $R^1$ | $R^2$ | $R^3$ | $R^4$ | $R^5$ | $OR^6$ | HX | Schmp. (°C) |
|---|---|---|---|---|---|---|---|---|---|
| 12 | 4-Cl | Me | H | H | Me | H | 4-OH | HBr | 272 (Z) |
| 13 | 4-Cl | Me | H | H | H | H | 4-OH | HBr | 274 (Z) |
| 14 | 4-Cl | Me | H | H | Me | H | 4-OH | – | 128–130 |
| 15 | 6-Cl | Me | H | 5-Me | Me | Me | 2-OH | – | 185–189 |
| 16 | 4-Cl | Me | H | 5-Me | H | H | 2-OH | HBr | 278–282 (Z) |
| 17 | 6-Cl | Me | H | 5-Me | Me | Me | 2-OH | HBr | 254–256 (Z) |
| 18 | 4-Cl | Me | H | 5-Me | Me | Me | 2-OH | – | 188–192 |
| 19 | 4-Cl | Me | H | 5-Me | Me | Me | 2-OH | HBr | 255–259 (Z) |
| 20 | 4-Cl | Me | H | H | n-Bu | H | 4-OH | – | 122–125 |
| 21 | 4-Cl | Me | H | H | n-Bu | H | 4-OH | HBr | 285 (Z) |
| 22 | 4-Cl | Me | H | H | n-Pr | H | 4-OH | – | 127–131 (Z) |
| 23 | 4-Cl | Me | H | H | n-Pr | H | 4-OH | – | 298–300 (Z) |

Fortsetzung

| Beisp.-Nr. | Y | $R^1$ | $R^2$ | $R^3$ | $R^4$ | $R^5$ | $OR^6$ | HX | Schmp. (°C) |
|---|---|---|---|---|---|---|---|---|---|
| 24 | 4-Cl | Me | H | 2-Me | H | H | 5-OH | – | 188–191 |
| 25 | 4-Cl | Me | H | 2-Me | H | H | 5-OH | HBr | 282 (Z) |
| 26 | 4-Cl | Me | H | H | Et | H | 4-OH | – | 126–129 (Z) |
| 27 | 4-Cl | Me | H | H | Et | H | 4-OH | HBr | 277–280 (Z) |
| 28 | 4-Cl | Et | H | H | Et | Et | 4-OH | – | 166–170 |
| 29 | 4-Cl | Et | H | H | Et | Et | 4-OH | HBr | 265 (Z) |
| 30 | 4-Cl | Et | H | H | Me | Me | 4-OH | – | 230–250 |
| 31 | 4-Cl | Et | H | H | Me | Me | 4-OH | HBr | 130–160 (Z) |
| 32 | 4-Cl | Me | H | H | H | H | 3-OH | – | 206–208 |
| 33 | 4-Cl | Me | H | H | H | H | 3-OH | HBr | 277 (Z) |
| 34 | 4-Cl | Me | 3-Cl | 5-Cl | H | H | 4-OH | – | 235–236 (Z) |
| 35 | 4-Cl | Me | 3-Cl | 5-Cl | H | H | 4-OH | HBr | 291 (Z) |
| 36 | 4-Cl | Me | H | H | H | 2-Bu | 4-OH | – | 125 (Z) |
| 37 | 4-Cl | Me | H | H | H | 2-Bu | 4-OH | HBr | 276 (Z) |
| 38 | 4-Cl | Me | H | H | Et | Et | 4-OH | – | 235–237 |
| 39 | 4-Cl | Me | H | H | Et | Et | 4-OH | HBr | 273 (Z) |
| 40 | 4-Cl | Me | H | 2-Cl | Me | Me | 4-OH | – | 197–200 |
| 41 | 4-Cl | Me | H | 2-Cl | Me | Me | 4-OH | HBr | 264 (Z) |
| 42 | 6-Cl | Me | H | 2-Cl | Me | Me | 4-OH | – | 230–235 |
| 43 | 6-Cl | Me | H | 2-Cl | Me | Me | 4-OH | HBr | 264 (Z) |
| 44 | 6-Cl | Me | H | 2-Me | Me | Me | 5-OH | – | 246–248 |
| 45 | 6-Cl | Me | H | 2-Me | Me | Me | 5-OH | HBr | 265–268 (Z) |
| 46 | 4-Cl | Me | H | 2-Cl | Et | Et | 4-OH | – | 220–223 |
| 47 | 4-Cl | Me | H | 2-Cl | Et | Et | 4-OH | HBr | 267 (Z) |
| 48 | 4-Cl | Me | H | 2-Cl | H | Me | 4-OH | – | 112–115 |
| 49 | 4-Cl | Me | H | 2-Cl | H | Me | 4-OH | HBr | 279–281 (Z) |
| 50 | 5-Cl | Me | H | 2-Cl | Me | Me | 4-OH | – | 133–140 (Z) |
| 51 | 5-Cl | Me | H | 2-Cl | Me | Me | 4-OH | HBr | 247 (Z) |
| 52 | 4-Cl | Me | H | H | H | H | 2-OH | – | 206–208 |
| 53 | 4-Cl | Me | H | H | H | H | 2-OH | HBr | 268–269 (Z) |

Fortsetzung

| Beisp.-Nr. | Y | $R^1$ | $R^2$ | $R^3$ | $R^4$ | $R^5$ | $OR^6$ | HX | Schmp. (°C) |
|---|---|---|---|---|---|---|---|---|---|
| 54 | 4-Br | Me | H | H | H | H | 4-OH | – | 209–211 |
| 55 | 4-Br | Me | H | H | H | H | 4-OH | HBr | 254 (Z) |
| 56 | 4-Cl | Me | H | 2-Me | Me | Me | 5-OH | – | 169–172 (Z) |
| 57 | 4-Cl | Me | H | 2-Me | Me | Me | 5-OH | HBr | 270–272 (Z) |
| 58 | 4-Cl | Me | H | 3-Me | Me | Me | 4-OH | – | 208–211 |
| 59 | 4-Cl | Me | H | 3-Me | Me | Me | 4-OH | HBr | 267–270 (Z) |
| 60 | 6-Cl | Me | H | H | Me | Me | 4-OH | HBr | 302–305 (Z) |
| 61 | 6-Cl | Me | H | H | Me | Me | 2-OH | – | 194–195 |
| 62 | 6-Cl | Me | H | H | Me | Me | 2-OH | HBr | 237 (Z) |
| 63 | 4-Cl | Me | H | H | Me | Me | 2-OH | – | 203–204 |
| 64 | 4-Cl | Me | H | H | Me | Me | 2-OH | HBr | 252 (Z) |
| 65 | 4-Cl | Me | H | 2-Cl | H | H | 4-OH | – | 252–255 (Z) |
| 66 | 4-Cl | Me | H | 2-Cl | H | H | 4-OH | HBr | 270 (Z) |
| 67 | 4-Cl | Me | H | H | Me | Me | 3-OH | ($xH_2O$) | 129 (Z) |
| 68 | 4-Cl | Me | H | H | Me | Me | 3-OH | HBr | 267 (Z) |
| 69 | 5-Cl | Me | H | H | Me | Me | 2-OH | – | 191–192 |
| 70 | 5-Cl | Me | H | H | Me | Me | 2-OH | HBr | 258 (Z) |
| 71 | 5-Cl | Me | H | 2-Me | Me | Me | 5-OH | HBr | 247–250 (Z) |
| 72 | 5-Cl | Et | H | H | Me | Me | 4-OH | HBr | 223–230 (Z) |
| 73 | 6-Cl | Et | H | H | Me | Me | 4-OH | HBr | 267–270 (Z) |
| 74 | 4-Cl | Me | H | H | Me | Me | 4-OH | TsOH | 246–248 (Z) |
| 75 | 4-Cl | Me | H | H | Me | Me | 4-OH | MsOH | 270–272 |
| 76 | 4-Br | Me | H | H | H | H | 4-OH | – | 209–211 |
| 77 | 4-Br | Me | H | H | H | H | 4-OH | HBr | 254 (Z) |
| 78 | 4-Cl | Me | 3-Cl | 5-Cl | Me | Me | 4-OH | HBr | 330 |
| 79 | 4-Cl | Me | H | 3-OMe | Me | Me | 4-OH | ($xH_2O$) | 163–166 |
| 80 | 4-Cl | Me | H | 3-OMe | Me | Me | 4-OH | HBr | 255–257 (Z) |
| 81 | 4-Cl | Me | H | 4-i-Pr | Me | Me | 3-OH | – | 108–115 (Z) |
| 82 | 4-Cl | Me | H | 4-i-Pr | Me | Me | 3-OH | HBr | 269 (Z) |
| 83 | 5-Me | Me | H | H | Me | Me | 4-OH | HBr | 310–316 (Z) |

Fortsetzung

| Beisp.-Nr. | Y | $R^1$ | $R^2$ | $R^3$ | $R^4$ | $R^5$ | $OR^6$ | HX | Schmp. (°C) |
|---|---|---|---|---|---|---|---|---|---|
| 84 | 6-Me | Me | H | H | Me | Me | 4-OH | – | 127–133 |
| 85 | 6-Me | Me | H | H | Me | Me | 4-OH | HBr | 293–296 (Z) |
| 86 | 4-Cl | Me | H | H | H | H | 4-OH | HCl | 270–272 (Z) |
| 87 | 4-Me | Me | H | H | Me | Me | 4-OH | – | 210 (Z) |
| 88 | 4-Cl | Et | H | H | H | H | 4-OH | HBr | 165–168 (Z) |
| 89 | 4-Cl | Me | H | 3-Me | H | H | 4-OH | HBr | 258–260 |
| 90 | 4-Cl | Me | H | 3-Me | H | H | 4-OH | (xEtOH) | 134–137 (Z) |
| 91 | 4-Cl | Me | H | 3-Cl | H | H | 4-OH | – | 210–225 (Z) |
| 92 | 4-Cl | Me | H | 3-Cl | H | H | 4-OH | HBr | 293 (Z) |
| 93 | 4-Cl | Me | 3-Cl | 4-Me | H | H | 2-OH | HBr | 273 (Z) |
| 94 | 4-Cl | Me | 3-Cl | 4-Me | H | H | 2-OH | – | 188–190 |
| 95 | 4-Cl | Me | 3-t-Bu | 5-t-Bu | H | H | 4-OH | – | 239–240 |
| 96 | 4-Cl | Me | 3-t-Bu | 5-t-Bu | Me | Me | 4-OH | – | 228–229 |
| 97 | 4-Cl | Me | 3-t-Bu | 5-t-Bu | H | H | 4-OH | HBr | 207 (Z) |
| 98 | 4-Cl | Me | 3-t-Bu | 5-t-Bu | Me | Me | 4-OH | HBr | 238 (Z) |
| 99 | 4-Cl | Me | 3-Me | 5-Me | Me | Me | 4-OH | – | 188–189 |
| 100 | 4-Cl | Me | 3-Me | 5-Me | Me | Me | 4-OH | HBr | 266 (Z) |
| 101 | 4-Cl | Me | 3-Me | 5-Me | H | H | 4-OH | HBr | 250 (Z) |
| 102 | 4-Cl | Me | 3-Me | 5-Me | H | H | 4-OH | – | 233–234 |
| 103 | 4-Cl | Me | H | H | $-(CH_2)_4-$ | | 4-OH | HBr | 276 (Z) |
| 104 | 4-Cl | Me | H | H | $-(CH_2)_4-$ | | 4-OH | – | 197–198 |

Tabelle 5

(Erklärungen vergl. Tabelle 3)

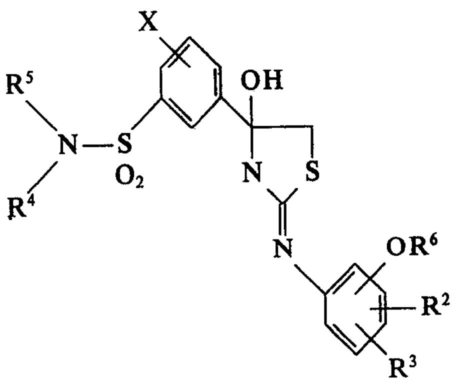

× HX

| Beisp.-Nr. | Y | $R^1$ | $R^2$ | $R^3$ | $R^4$ | $R^5$ | $OR^6$ | HX | Schmp. (°C) |
|---|---|---|---|---|---|---|---|---|---|
| 105 | 4-Cl | Me | 3-Cl | 5-Cl | H | H | 4-OH | HBr | 286 (Z) |
| 106 | 4-Cl | Me | H | 3-Cl | H | H | 4-OH | HBr | 199 (Z) |
| 107 | 4-Cl | Me | H | H | H | H | 3-OH | HBr | 152 (Z) |
| 108 | 4-Cl | Me | H | H | H | 2-Bu | 4-OH | HBr | 277 (Z) |
| 109 | 4-Cl | Me | H | H | Et | Et | 4-OH | HBr | 276 (Z) |
| 110 | 4-Cl | Me | 3-Cl | 5-Cl | Me | Me | 4-OH | HBr | 264 (Z) |
| 111 | 4-Cl | Me | H | H | Me | Me | 3-OH | HBr | 263 (Z) |
| 112 | 4-Cl | Me | 4-Me | 5-Cl | H | H | 2-OH | HBr | 276 (Z) |
| 113 | 4-Cl | Me | H | H | H | H | 2-OH | HBr | 182 (Z) |
| 114 | 5-Cl | Me | H | H | Me | Me | 4-OH | HBr | 282 (Z) |
| 115 | 5-Cl | Me | H | H | Me | Me | 2-OH | HBr | 248 (Z) |
| 116 | 6-Cl | Me | H | H | Me | Me | 2-OH | HBr | 198 (Z) |
| 177 | 5-Cl | Me | H | H | Me | Me | 2-OH | HBr | 261 (Z) |
| 118 | 4-Cl | Me | H | H | H | H | 4-OH | HBr | 170 (Z) |
| 119 | 4-Cl | Me | H | H | Me | H | 4-OH | HBr | 155/267 (Z) |
| 120 | 4-Cl | Me | H | H | Me | Me | 4-OH | – | 121 (Z) |
| 121 | 4-Cl | Me | H | H | H | Me | 4-OH | – | 142 (Z) |
| 122 | 4-Cl | Me | H | H | H | H | 4-OH | – | 174 (Z) |
| 123 | 4-Cl | Me | H | 5-Me | H | H | 2-OH | HBr | 185/220 (Z) |
| 124 | 4-Cl | Et | H | H | H | H | 4-OH | HBr | 175 (Z) |
| 125 | 4-Cl | Me | 4-Me | 5-Me | H | H | 2-OH | HBr | 220–5 (Z) |
| 126 | 6-Cl | Me | 5-Me | H | Me | Me | 2-OH | HBr | 255 (Z) |

Forsetzung

| Beisp.-Nr. | Y | $R^1$ | $R^2$ | $R^3$ | $R^4$ | $R^5$ | $OR^6$ | HX | Schmp. (°C) |
|---|---|---|---|---|---|---|---|---|---|
| 127 | 4-Cl | Me | H | 3-Me | H | H | 4-OH | HBr | 188–92 (Z) |
| 128 | 4-Cl | Me | H | 2-Cl | H | H | 4-OH | HBr | 156 (Z) |
| 129 | 4-Cl | Me | H | 5-Me | Me | Me | 4-OH | HBr | 260 (Z) |
| 130 | 4-Cl | Me | H | H | n-Bu | H | 4-OH | HBr | 187–90 (Z) |
| 131 | 4-Cl | Me | H | H | Et | H | 4-OH | HBr | 175–178 (Z) |
| 132 | 4-Cl | Me | H | H | n-Pr | H | 4-OH | HBr | 183–5 (Z) |
| 133 | 4-Cl | Et | H | H | Me | Me | 4-OH | HBr | 190 (Z) |
| 134 | 4-Cl | Me | 2-Me | H | H | H | 5-OH | HBr | 189–99 (Z) |
| 135 | 4-Cl | Et | H | H | Et | Et | 4-OH | HBr | 263–5 (Z) |
| 136 | 4-Cl | Me | 2-Cl | H | Me | Me | 4-OH | HBr | 276 (Z) |
| 137 | 6-Cl | Me | 2-Cl | H | Me | Me | 4-OH | HBr | 210–4 (Z) |
| 138 | 6-Cl | Me | 2-Me | H | Me | Me | 5-OH | HBr | 198–201 (Z) |
| 139 | 4-Cl | Me | 2-Me | H | Me | Me | 5-OH | HBr | 266 (Z) |
| 140 | 4-Cl | Me | 2-Cl | H | Et | Et | 4-OH | HBr | 266 (Z) |
| 141 | 4-Cl | Me | 2-Cl | H | Me | H | 4-OH | HBr | 280 (Z) |
| 142 | 5-Cl | Me | 2-Cl | H | Me | Me | 4-OH | HBr | 218–21 (Z) |
| 143 | 4-Cl | Me | 2-Me | H | Me | Me | 4-OH | HBr | 268 (Z) |
| 144 | 6-Cl | Me | H | H | Me | Me | 4-OH | HBr | 298 (Z) |
| 145 | 5-Cl | Et | H | H | Me | Me | 4-OH | HBr | 130 (Z) |
| 146 | 4-Br | Me | H | H | H | H | 4-OH | HBr | 168 (Z) |
| 147 | 4-Me | Me | H | H | Me | Me | 4-OH | HBr | 220–40 (Z) |
| 148 | 6-Me | Me | H | H | Me | Me | 4-OH | HBr | 180 (Z) |
| 149 | 5-Me | Me | H | H | Me | Me | 4-OH | HBr | 310 (Z) |

Tabelle 6

Tabelle 6 zeigt einige der hergestellten Thioharnstoffe III, die nach literaturbekannten Methoden gewonnen werden (vergl. Seite 6)

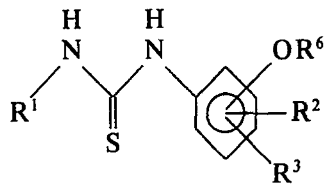

(III)

(Abkürzungen wie in Tabelle 3!)

| $R^1$ | $R^2$ | $R^3$ | $OR^6$ | Schmp. (°C) |
|---|---|---|---|---|
| Me | H | H | 4-OH | 195–196 |
| Me | 5-Me | H | 2-OH | 176–179 |
| Et | H | H | 4-OH | 152–155 |
| Me | 3-Me | H | 4-OH | 233–236 |
| Me | 2-Cl | H | 4-OH | 164–167 |
| Me | 4-Me | 5-Me | 2-OH | 158–162 |
| Me | 2-Me | H | 5-OH | 202–205 |
| Me | H | H | 3-OH | 165–167 |
| Me | H | H | 2-OH | 123–126 |
| Me | 3-Cl | H | 4-OH | 216–218 |
| Me | 3-Cl | 5-Cl | 4-OH | 204–206 |
| Me | 3-t-Bu | 5-t-Bu | 4-OH | 198–200 |
| Me | 3-Me | 5-Me | 4-OH | 200–201 |
| Me | 3-OMe | H | 4-OH | 193–195 |
| Me | 4-i-Pr | H | 3-OH | 145–147 |

**Patentansprüche für die Vertragsstaaten BE, CH, DE, FR, GB, IT, LI, LU, NL, SE**

1. Thiazolinderivate der allgemeinen Formel I

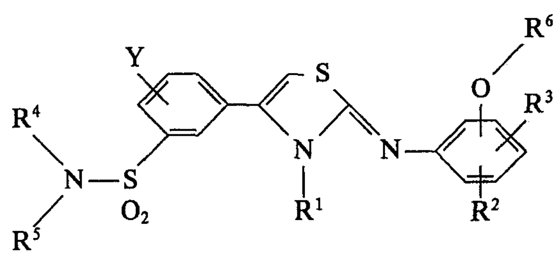

(I)

in welcher

$R^1$ Alkyl mit 1 bis 3 C-Atomen,

$R^2$ und $R^3$ Wasserstoff, Halogen, Alkyl oder Alkoxy mit jeweils 1 bis 4 C-Atomen, wobei $R^2$ und $R^3$ gleich oder verschieden sind,

$R^4$ und $R^5$ Wasserstoff oder Alkyl mit 1 bis 4 C-Atomen, wobei $R^4$ und $R^5$ gleich oder verschieden sind oder zusammen mit dem N-Atom einen gesättigten Ring mit bis zu 6 Ringgliedern bilden,

$R^6$ Wasserstoff oder Acyl mit 1 bis 4 C-Atomen und

Y Wasserstoff, Halogen oder Methyl

bedeuten, sowie deren physiologisch verträglichen Salze.

2. Thiazolinderivate der in Anspruch 1 angegebenen allgemeinen Formel I, worin

$R^1$ Methyl oder Ethyl,
$R^2$ Wasserstoff oder Chlor,
$R^3$ Wasserstoff oder Chlor oder Methyl,
$R^4$ Wasserstoff oder Methyl oder Ethyl,
$R^5$ Wasserstoff oder Methyl,
$R^6$ Wasserstoff oder $-CO-CH_3$ oder $-COC_2H_5$,
Y Chlor, Brom oder Methyl in 4-, 5- oder 6-Stellung zum Thiazolin-Ring (wobei der Sulfamoylrest in 3-Stellung festgelegt ist)

bedeuten, sowie deren physiologisch verträgliche Salze.

3. Thiazolinderivate der in Anspruch 1 angegebenen allgemeinen Formel I, in welcher

$R^1$ Methyl,
$R^2$ und $R^3$ Wasserstoff,
$R^4$ Wasserstoff oder Methyl,
$R^5$ Wasserstoff oder Methyl,
$R^6$ Wasserstoff, wobei die OH-Gruppe in 4-Stellung zur Iminogruppe steht,
Y Chlor in 4-Stellung zum Thiazolin,

bedeuten, sowie deren physiologisch verträglichen Salze.

4. 4-(4-Chlor-3-sulfamoylphenyl)-2-(4-hydroxyphenylimino)-3-methyl-4-thiazolin gemäß Anspruch 1 sowie dessen pharmakologisch verträglichen Säureadditionssalze.

5. 4-(4-Chlor-3-methylsulfamoylphenyl)-2-(4-hydroxyphenylimino)-3-methyl-4-thiazolin gemäß Anspruch 1 sowie dessen pharmakologisch verträglichen Säureadditionssalze.

6. 4-(4-Chlor-3-dimethylsulfamoylphenyl)-2-(4-hydroxyphenylimino)-3-methyl-4-thiazolin gemäß Anspruch 1 sowie dessen pharmakologisch verträgliche Säureadditionssalze.

7. Verfahren zur Herstellung von Thiazolinderivaten gemäß Anspruch 1, dadurch gekennzeichnet, daß man

a) Verbindungen der allgemeinen Formel II

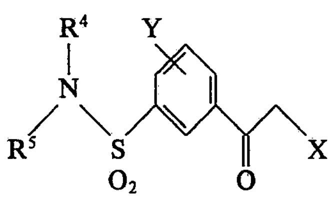

(II)

worin $R^4$, $R^5$ und Y die angegebene Bedeutung besitzen, X eine leaving group ist unter kondensierenden Reaktionsbedingungen mit einem Thioharnstoff der allgemeinen Formel III

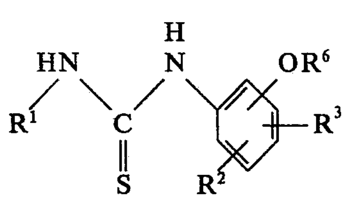

(III)

worin $R^1$, $R^2$, $R^3$, $R^6$ die angegebene Bedeutung besitzen, umsetzt, oder

b) aus Verbindungen der allgemeinen Formel IV

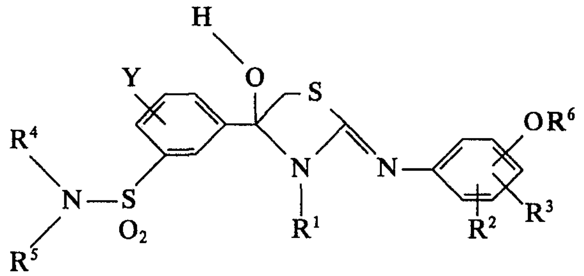

(IV)

worin $R^1$ bis $R^6$ und Y die angegebene Bedeutung besitzen, Wasser abspaltet, oder

c) Verbindungen der allgemeinen Formel V

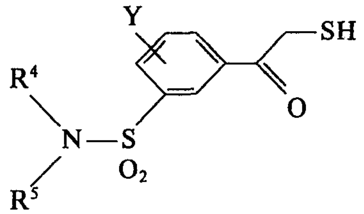

(V)

mit Verbindungen der allgemeinen Formel VI

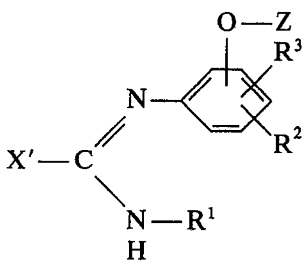

(VI)

zur Reaktion bringt, wobei $R^1$ bis $R^5$ die angegebene Bedeutung haben, Z eine Schutzgruppe der phenolischen Hydroxylgruppe bedeutet und X' eine leaving group ist, oder

d) Verbindungen der allgemeinen Formel VII

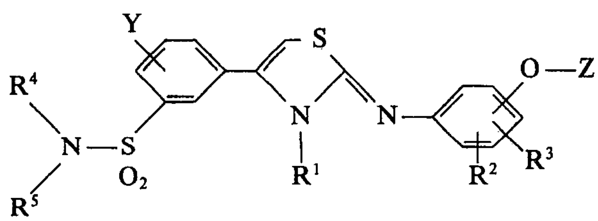

(VII)

worin $R^1$ bis $R^5$ und Y die zu Formel I und Z die zu Formel VI angegebene Bedeutung besitzen, durch eine hydrolytische oder solvolytische Spaltung in die Verbindungen der Formel I überführt, worin $R^6$ die Bedeutung von Wasserstoff hat, oder

e) Verbindungen der allgemeinen Formel I, worin $R^1$ bis $R^5$ und Y die angegebene Bedeutung besitzen und $R^6$ für Wasserstoff steht, mit einem Acylierungsmittel in Verbindungen der Formel I mit $R^6$ in der Bedeutung von Acyl mit 1 bis 4 C-Atomen überführt, und gegebenenfalls eine der nach Weg a) bis e) erhaltenen Verbindungen der allgemeinen Formel I mit organischen oder anorganischen Säuren der allgemeinen Formel H—A in ihre Säureadditionssalze oder erhaltene Salze der Verbindungen der allgemeinen Formel I mit Basen in die freien basischen Verbindungen der Formel I überführt, oder eine erhaltene Verbindung der Formel I, worin $R^6$ Wasserstoff bedeutet, mit organischen oder anorganischen Basen in Phenolatsalze oder entsprechend erhaltene Salze mit Säuren in die freien basischen Verbindungen der Formel I überführt.

8. Pharmazeutisches Präparat auf Basis einer Verbindung gemäß Anspruch 1.

9. Verfahren zur Herstellung eines pharmazeutischen Präparates gemäß Anspruch 8, dadurch gekennzeichnet, daß man eine Verbindung gemäß Anspruch 1 gegebenenfalls mit pharmakologisch verträglichen Trägern und/oder Stabilisatoren in eine geeignete Darreichungsform überführt.

10. Eine Verbindung gemäß Anspruch 1 zur Verwendung bei der Behandlung von Störungen des Serumlipoproteinspektrums.

11. Verbindungen der allgemeinen Formel IV

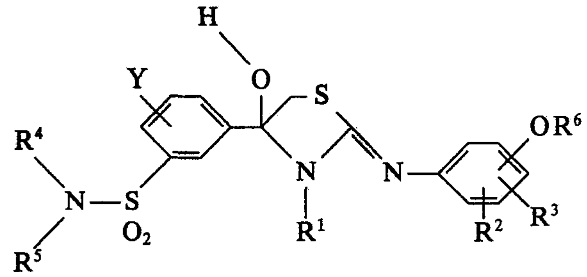

(IV)

worin $R^1$ bis $R^6$ und Y die zu Formel I angegebenen Bedeutungen besitzen, sowie deren Salze.

**Patentansprüche für den Vertragsstaat AT**

1. Verfahren zur Herstellung von Thiazolinderivaten der allgemeinen Formel I

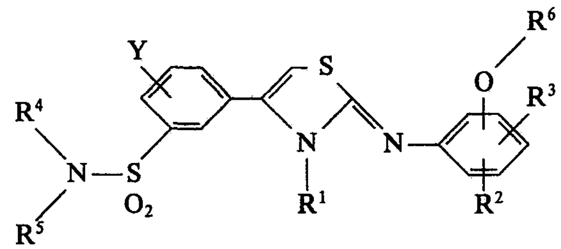

(I)

in welcher

| | |
|---|---|
| $R^1$ | Alkyl mit 1 bis 3 C-Atomen, |
| $R^2$ und $R^3$ | Wasserstoff, Halogen, Alkyl oder Alkoxy mit jeweils 1 bis 4 C-Atomen, wobei $R^2$ und $R^3$ gleich oder verschieden sind, |
| $R^4$ und $R^5$ | Wasserstoff oder Alkyl mit 1 bis 4 C-Atomen, wobei $R^4$ und $R^5$ gleich oder verschieden sind oder zusammen mit dem N-Atom einen gesättigten Ring mit bis zu 6 Ringgliedern bilden, |
| $R^6$ | Wasserstoff oder Acyl mit 1 bis 4 C-Atomen und |
| Y | Wasserstoff, Halogen oder Methyl |

bedeuten, sowie von deren physiologisch verträglichen Salzen, dadurch gekennzeichnet, daß man

a) Verbindungen der allgemeinen Formel II

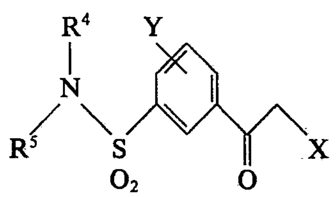

(II)

worin $R^4$, $R^5$ und Y die angegebene Bedeutung besitzen, X eine leaving group ist unter kondensierenden Reaktionsbedingungen mit einem Thioharnstoff der allgemeinen Formel III

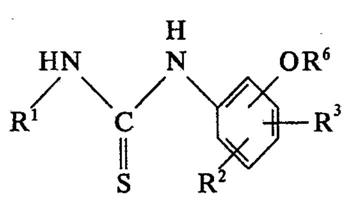

(III)

worin $R^1$, $R^2$, $R^3$, $R^6$ die angegebene Bedeutung besitzen, umsetzt, oder

b) aus Verbindungen der allgemeinen Formel IV

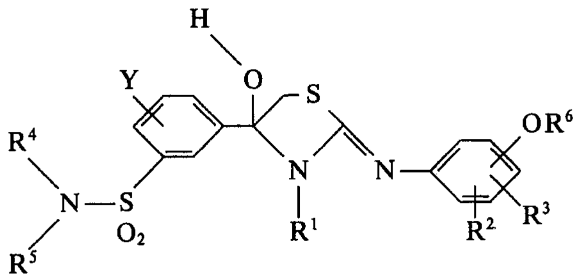

(IV)

worin $R^1$ bis $R^6$ und Y die angegebene Bedeutung besitzen, Wasser abspaltet, oder

c) Verbindungen der allgemeinen Formel V

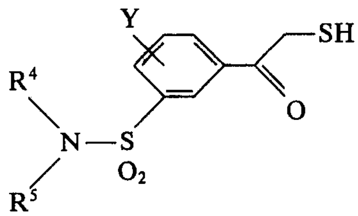

(V)

mit Verbindungen der allgemeinen Formel VI

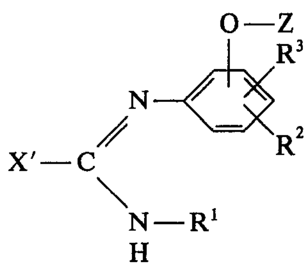

(VI)

zur Reaktion bringt, wobei $R^1$ bis $R^5$ die angegebene Bedeutung haben, Z eine Schutzgruppe der phenolischen Hydroxylgruppe bedeutet und X' eine leaving group ist, oder

d) Verbindungen der allgemeinen Formel VII

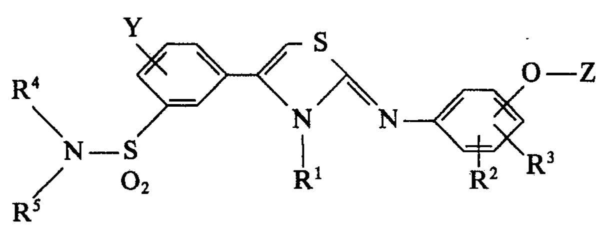

(VII)

worin $R^1$ und $R^5$ und Y die zu Formel I und Z die zu Formel VI angegebene Bedeutung besitzen, durch eine hydrolytische oder solvolytische Spaltung in die Verbindungen der Formel I überführt, worin $R^6$ die Bedeutung von Wasserstoff hat, oder

e) Verbindungen der allgemeinen Formel I, worin $R^1$ bis $R^5$ und Y die angegebene Bedeutung besitzen und $R^6$ für Wasserstoff steht, mit einem Acylierungsmittel in Verbindungen der Formel I mit $R^6$ in der Bedeutung von Acyl mit 1 bis 4 C-Atomen überführt, und gegebenenfalls eine der nach Weg a) bis e) erhaltenen Verbindungen der allgemeinen Formel I mit organischen oder anorganischen Säuren der allgemeinen Formel H—A in ihre Säureadditionssalze oder erhaltene Salze der Verbindungen der allgemeinen Formel I mit Basen in die freien basischen Verbindungen der Formel I überführt, oder eine erhaltene Verbindung der Formel I, worin $R^6$ Wasserstoff bedeutet, mit organischem oder anorganischen Basen in Phenolatsalze oder entsprechend erhaltene Salze mit Säuren in die freien basischen Verbindungen der Formel I überführt.

2. Verfahren gemäß Anspruch 1, dadurch gekennzeichnet, daß man Thiazolinderivate der in Anspruch 1 angegebenen allgemeinen Formel I, worin

$R^1$ Methyl oder Ethyl,
$R^2$ Wasserstoff oder Chlor,
$R^3$ Wasserstoff oder Chlor oder Methyl,

$R^4$ Wasserstoff oder Methyl oder Ethyl,
$R^5$ Wasserstoff oder Methyl,
$R^6$ Wasserstoff oder $-COCH_3$ oder $-COC_2H_5$
Y Chlor, Brom oder Methyl in 4-, 5- oder 6-Stellung zum Thiazolin-Ring (wobei der Sulfamoylrest in 3-Stellung festgelegt ist)

bedeuten, oder deren physiologisch verträgliche Salze herstellt.

3. Verfahren gemäß Anspruch 1, dadurch gekennzeichnet, daß man Thiazolinderivate der in Anspruch 1 angegebenen allgemeinen Formel I, in welcher

$R^1$ Methyl,
$R^2$ und $R^3$ Wasserstoff,
$R^4$ Wasserstoff oder Methyl,
$R^5$ Wasserstoff oder Methyl,
$R^6$ Wasserstoff, wobei die OH-Gruppe in 4-Stellung zur Iminogruppe steht,
Y Chlor in 4-Stellung zum Thiazolin,

bedeuten, oder deren physiologisch verträglichen Salze herstellt.

4. Verfahren gemäß Anspruch 1, dadurch gekennzeichnet, daß man 4-(4-Chlor-3-sulfamoylphenyl)-2-(4-hydroxyphenylimino)-3-methyl-4-thiazolin oder dessen pharmakologisch verträglichen Säureadditionssalze herstellt.

5. Verfahren gemäß Anspruch 1, dadurch gekennzeichnet, daß man 4-(4-Chlor-3-methylsulfamoylphenyl)-2-(4-hydroxyphenylimino)-3-methyl-4-thiazolin oder dessen pharmakologisch verträgliche Säureadditionssalze herstellt.

6. Verfahren gemäß Anspruch 1, dadurch gekennzeichnet, daß man 4-(4-Chlor-3-dimethylsulfamoylphenyl)-2-(4-hydroxyphenylimino)-3-methyl-4-thiazolin oder dessen pharmakologisch verträgliche Säureadditionssalze herstellt.

**Claims for the contracting states: BE, CH, DE, FR, GB, IT, LI, LU, NL, SE**

1. A thiazoline derivative of the general formula I

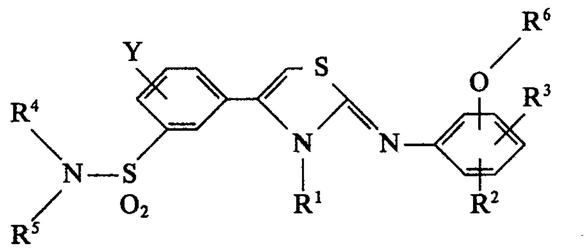

(I)

in which $R^1$ is alkyl with 1 to 3 C atoms, $R^2$ and $R^3$ are hydrogen, halogen, alkyl or alkoxy with in each case 1 to 4 C atoms, and are identical or different, $R^4$ and $R^5$ are hydrogen or alkyl with 1 to 4 C atoms, and are identical or different or together with the N atom form a saturated ring with up to 6 ring members, $R^6$ is hydrogen or acyl with 1 to 4 C atoms and Y is hydrogen, halogen or methyl, as well as its physiologically tolerated salts.

2. A thiazoline derivative of the general formula I given in claim 1, wherein $R^1$ is methyl or ethyl, $R^2$ is hydrogen or chlorine, $R^3$ is hydrogen or chlorine or methyl, $R^4$ is hydrogen or methyl or ethyl, $R^5$ is hydrogen or methyl, $R^6$ is hydrogen or $-CO-CH_3$ or $-COC_2H_5$, Y is chlorine, bromine or methyl in the 4,5- or 6-position to the thiazoline ring (the sulfamoyl radical being fixed in the 3-position), as well as its physiologically tolerated salts.

3. A thiazoline derivative of the general formula I given in claim 1, in which $R^1$ is methyl, $R^2$ and $R^3$ are hydrogen, $R^4$ is hydrogen or methyl, $R^5$ is hydrogen or methyl, $R^6$ is hydrogen, the OH groups being in the 4-position to the imino group and Y is chlorine in the 4-position to the thiazoline, as well as its physiologically tolerated salts.

4. 4-(4-Chloro-3-sulfamoylphenyl)-2-(4-hydroxyphenylimino)-3-methyl-4-thiazoline as claimed in claim 1 as well as its pharmacologically tolerated acid addition salts.

5. 4-(4-Chloro-3-methylsulfamoylphenyl)-2-(4-hydroxyphenylimino)-3-methyl-4-thiazoline as claimed in claim 1 as well as its pharmacologically tolerated acid addition salts.

6. 4-(4-Chloro-3-dimethylsulfamoylphenyl)-2-(4-hydroxyphenylimino)-3-methyl-4-thiazoline as claimed in claim 1 as well as its pharmacologically tolerated acid addition salts.

7. A process for the preparation of a thiazoline derivative as claimed in claim 1, which comprises

a) reacting, under condensation conditions, a compound of the general formula II

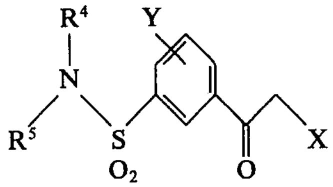

(II)

wherein $R^4$, $R^5$ and Y have the abovementioned meaning and X is a leaving group, with a thiourea of the general formula III

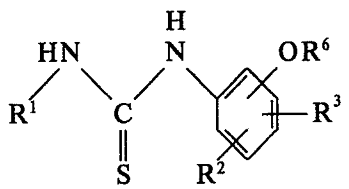

(III)

wherein $R^1$, $R^2$, $R^3$ and $R^6$ have the abovementioned meaning or

b) eliminating water from a compound of the general formula IV

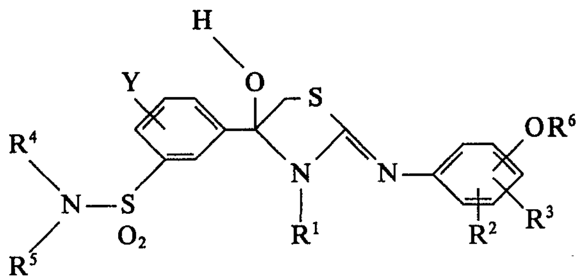

(IV)

wherein $R^1$ to $R^6$ and Y have the abovementioned meaning or

c) reacting a compound of the general formula V

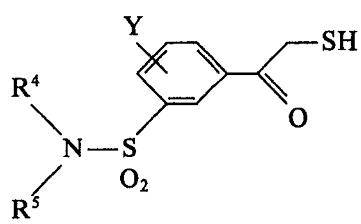

(V)

with a compound of the general formula VI

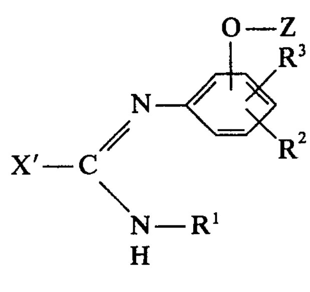

(VI)

wherein $R^1$ to $R^5$ have the abovementioned meaning, Z is a protective group for the phenolic hydroxyl group and X' is a leaving group or

d) converting, by hydrolytic or solvolytic cleavage, a compound of the general formula VII

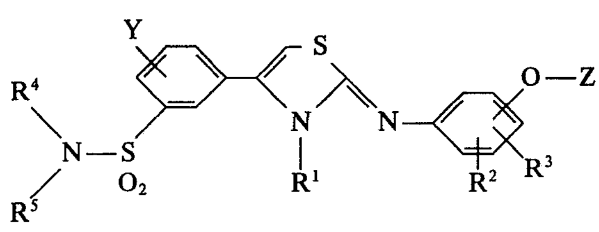

(VII)

wherein $R^1$ to $R^5$ and Y have the meanings given for formula I and Z has the meaning given for formula VI, into a compound of the formula I, wherein $R^6$ is hydrogen, or

e) converting a compound of the general formula I, wherein $R^1$ to $R^5$ and Y have the abovementioned meaning and $R^6$ is hydrogen, by means of an acylating agent, into a compound of the formula I, with $R^6$ having the meaning of acyl with 1 to 4 C atoms, and if desired, converting one of the compounds of the general formula I obtained by one of the routes a) to e), into its acid addition salt, by means of an organic or inorganic acid of the general formula H—A, or converting a resulting salt of the compound I, by means of a base, into the free basic compound of the formula I, or converting a resulting compound of the formula I, wherein $R^6$ is hydrogen, by means of an organic or inorganic base into a phenolate salt or converting a correspondingly obtained salt by means of an acid into the free basic compound of the formula I.

8. A pharmaceutical preparation based on a compound as claimed in claim 1.

9. A process for the preparation of a pharmaceutical preparation as claimed in claim 8, which comprises converting a compound as claimed in claim 1, if appropriate with pharmacologically tolerated carriers and/or stabilizers, into a suitable form for administration.

10. A compound according to claim 1 for the use in the treatment of disorders of the serum lipoprotein spectrum.

11. A compound of the general formula IV

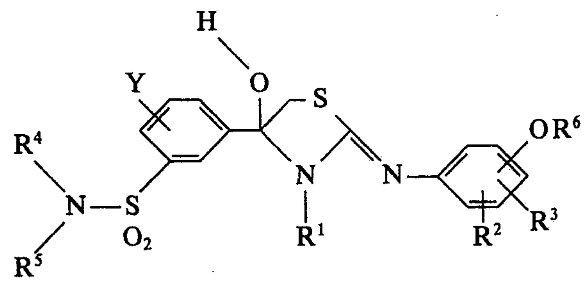

(IV)

wherein $R^1$ to $R^6$ and Y have the meanings given for formula I, as well as its salts.

**Claims for the contracting state: AT**

1. A process for the preparation of a thiazoline derivative of the general formula I

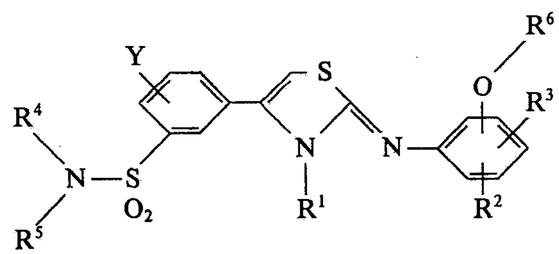

(I)

in which $R^1$ is alkyl with 1 to 3 C atoms, $R^2$ and $R^3$ are hydrogen, halogen, alkyl or alkoxy with in each case 1 to 4 C atoms, and are identical or different, $R^4$ and $R^5$ are hydrogen or alkyl with 1 to 4 C atoms, and are identical or different or together with the N atom form a saturated ring with up to 6 ring members, $R^6$ is hydrogen or acyl with 1 to 4 C atoms and Y is hydrogen, halogen or methyl, as well as of its physiologically tolerated salts, which comprises

a) reacting, under condensation conditions, a compound of the general formula II

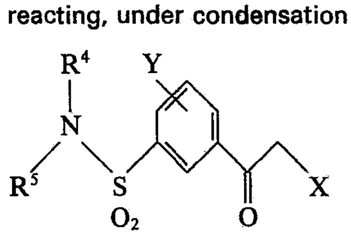

(II)

wherein $R^1$, $R^1$ and Y have the abovementioned meaning and X is a leaving group, with a thiourea of the general formula III

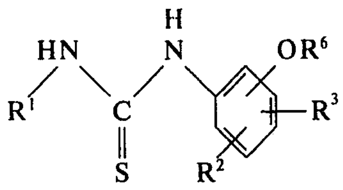

(III)

wherein $R^1$, $R^2$, $R^3$ and $R^6$ have the abovementioned meaning or

b) eliminating water from a compound of the general formula IV

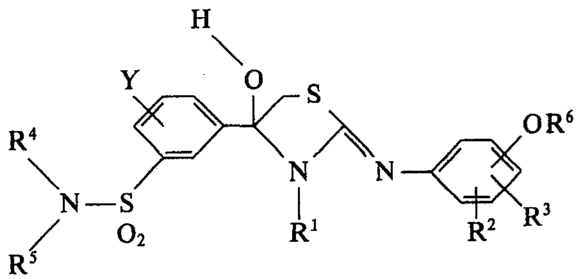

(IV)

wherein $R^1$ to $R^6$ and Y have the abovementioned meaning or

c) reacting a compound of the general formula V

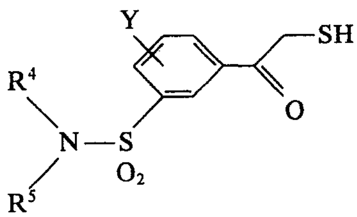

(V)

with a compound of the general formula VI

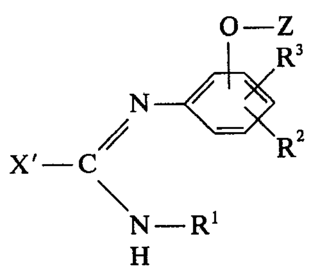

(VI)

wherein $R^1$ to $R^5$ have the abovementioned meaning, Z is a protective group for the phenolic hydroxyl group and X' is a leaving group or

d) converting, by hydrolytic or solvolytic cleavage, a compound of the general formula VII

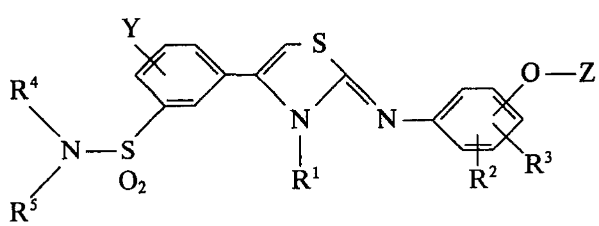

(VII)

wherein $R^1$ to $R^5$ and Y have the meanings given for formula I and Z has the meaning given for formula VI, into a compound of the formula I, wherein $R^6$ is hydrogen, or

e) converting a compound of the general formula I, wherein $R^1$ to $R^5$ and Y have the abovementioned meaning and $R^6$ is hydrogen, by means of an acylating agent, into a compound of the formula I with $R^6$ having the meaning of acyl with 1 to 4 C atoms, and if desired, converting one of the compounds of the general formula I obtained by one of the routes a) to e), into its acid addition salt, by means of an organic or inorganic acid of the general formula H—A, or converting a resulting salt of the compound of the general formula I, by means of a base, into the free basic compound of the formula I, or converting a resulting compound of the formula I, wherein $R^6$ is hydrogen, by means of an organic

or inorganic base into a phenolate salt, or converting a correspondingly obtained salt by means of an acid into the free basic compound of the formula I.

2. A process as claimed in claim 1 which comprises preparing a thiazoline derivative of the general formula I given in claim 1, wherein $R^1$ is methyl or ethyl, $R^2$ is hydrogen or chlorine, $R^3$ is hydrogen or chlorine or methyl, $R^4$ is hydrogen or methyl or ethyl, $R^5$ is hydrogen or methyl, $R^6$ is hydrogen or $-CO-CH_3$ or $-COC_2H_5$, Y is chlorine, bromine or methyl in the 4-, 5- or 6-position to the thiazoline ring (the sulfamoyl radical being fixed in the 3-position) or its physiologically tolerated salts.

3. A process as claimed in claim 1, which comprises preparing a thiazoline derivative of the general formula I given in claim 1, in which $R^1$ is methyl, $R^2$ and $R^3$ are hydrogen, $R^4$ is hydrogen or methyl, $R^5$ is hydrogen or methyl, $R^6$ is hydrogen, the OH group being in the 4-position to the imino group and Y is chlorine in the 4-position to the thiazoline, or its physiologically tolerated salts.

4. A process as claimed in claim 1, which comprises preparing 4-(4-chloro-3-sulfamoylphenyl)-2-(4-hydroxyphenylimino)-3-methyl-4-thiazoline, or its pharmacologically tolerated acid addition salts.

5. A process as claimed in claim 1, which comprises preparing 4-(4-chloro-3-methylsulfamoylphenyl)-2(4-hydroxyphenylimino)-3-methyl-4-thiazoline, or its pharmacologically tolerated acid addition salts.

6. A process as claimed in claim 1 which comprises preparing 4-(4-chloro-3-dimethylsulfamoylphenyl)-2-(4-hydroxyphenylimino)-3-methyl-4-thiazoline, or its pharmacologically tolerated acid addition salts.

**Revendications pour les Etats contractants: BE, CH, DE, FR, GB, IT, LI, LU, NL, SE**

1. Dérivés de thiazoline de formule générale I

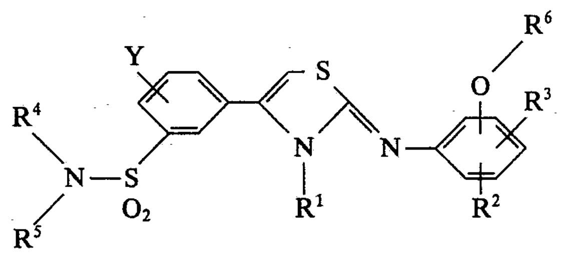

(I)

dans laquelle

| | |
|---|---|
| $R^1$ | désigne un groupe alkyle en $C_1$ à $C_3$, |
| $R^2$ et $R^3$ | désignent l'hydrogène, un halogène, un groupe alkyle ou alcoxy avec à chaque fois de 1 à 4 atomes de C, $R^2$ et $R^3$ étant identiques ou différents, |
| $R^4$ et $R^5$ | désignent l'hydrogène ou un groupe alkyle en $C_1$ à $C^4$, $R^4$ et $R^5$ étant identiques ou différents, ou forment avec l'atome de N un cycle saturé avec jusqu'à 6 chaînons dans le cycle, |
| $R^6$ | désigne l'hydrogène ou un group acyle en $C_1$ à $C_4$ et |
| Y | désigne l'hydrogène, un halogène ou un groupe méthyle, |

ainsi que leurs sels physiologiquement acceptables.

2. Dérivés de thiazoline de formule I indiquée dans la revendication 1, dans lesquels

$R^1$ désigne un groupe méthyle ou éthyle,
$R^2$ désigne l'hydrogène ou le chlore,
$R^3$ désigne l'hydrogène ou le chlore ou un groupe méthyle,
$R^4$ désigne l'hydrogène ou un groupe méthyle ou éthyle,
$R^5$ désigne l'hydrogène ou un groupe méthyle,
$R^6$ désigne l'hydrogène ou $-CO-CH_3$ ou $-COC_2H_5$,
Y désigne le chlore, le brome ou un groupe méthyle en position 4, 5 ou 6 par rapport au cycle thiazoline (le radical sulfamoyle étant fixé en position 3),

ainsi que leurs sels physiologiquement acceptables.

3. Dérivés de thiazoline de formule générale I indiquée dans la revendication I, dans lesquels:

| | |
|---|---|
| $R^1$ | désigne un groupe méthyle, |
| $R^2$ et $R^3$ | désignent l'hydrogène, |
| $R^4$ | désigne l'hydrogène ou un groupe méthyle, |
| $R^5$ | désigne l'hydrogène ou un groupe méthyle, |

$R^6$ désigne l'hydrogène, le groupe OH étant en position 4 par rapport au groupe imino,
Y désigne un chlore en position 4 par rapport à la thiazoline,

ainsi que leurs sels physiologiquement acceptables.

4. 4-(4-chloro-3-sulfamoylphényl)-2-(4-hydroxyphénylimino)-3-méthyl-4-thiazoline suivant la revendication 1 ainsi que ses sels d'addtition avec des acides pharmacologiquement acceptables.

5. 4-(4-chloro-3-méthylsulfamoylphényl)-2-(4-hydroxyphénylimino)-3-méthyl-4-thiazoline suivant la revendication 1 ainsi que ses sels d'addition avec des acides pharmacologiquement acceptables.

6. 4-(4-chloro-3-diméthylsulfamoylphényl)-2-(4-hydroxyphénylimino)-3-méthyl-4-thiazoline suivant la revendication 1 ainsi que ses sels d'addition avec des acides pharmacologiquement acceptables.

7. Procédé de préparation de dérivés de thiazoline suivant la revendication 1, caractérisé en ce que

a) on fait réagir des composés de formule générale II

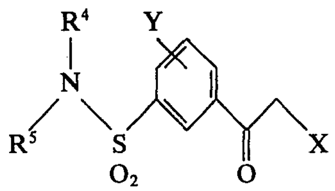

(II)

dans laquelle $R^4$, $R^5$ et Y ont la signification indiquée, X est un groupe qui part, dans des conditions de réaction condensantes, avec une thiourée de formule générale III

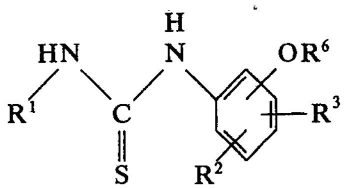

(III)

dans laquelle $R^1$, $R^2$, $R^3$, $R^6$ ont la signification indiquée, ou

b) à partir de composés de formule générale IV

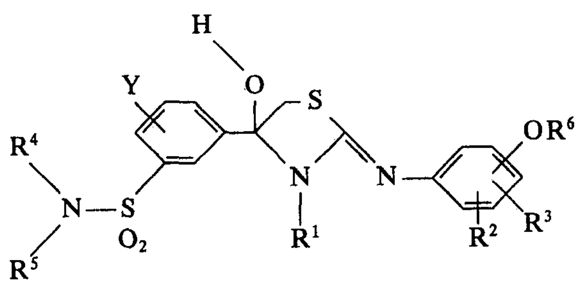

(IV)

dans laquelle $R^1$ à $R^6$ et Y ont la signification indiquée, on élimine de l'eau, ou

c) on fait réagir des composés de formule générale V

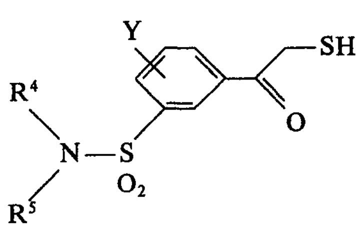

(V)

avec des composés de formule générale VI

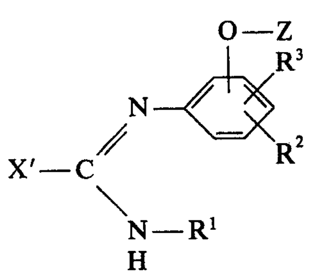

(VI)

$R^1$ à $R^5$ ayant la signification indiquée, Z désignant un groupe protecteur du groupe hydroxyle phénolique et X' étant un groupe qui part, ou

d) on transforme des composés de formule générale VII

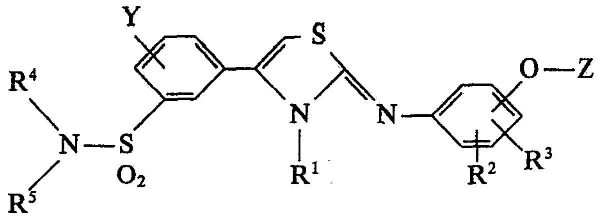

(VII)

dans laquelle $R^1$ à $R^5$ et Y ont la signification indiquée à propos de la formule I et Z a la signification indiquée à propos de la formule VI, par une scission hydrolytique ou solvolytique, en les composés de formule I, dans laquelle $R^6$ désigne l'hydrogène, ou

e) on transforme des composés de formule générale I, dans laquelle $R^1$ à $R^5$ et Y ont la signification indiquée et $R^6$ représente l'hydrogène, avec un agent d'acylation, en composés de formule I avec $R^6$ désignant un acyle en $C_1$ à $C_4$, et le cas échéant un des composés de formule générale I obtenus par les modes opératoires a) à e) est transformé avec des acides organiques ou minéraux de formule générale H—A en ses sels d'addition avec des acides ou les sels des composés de formule générale I obtenus sont transformés avec des bases en les composés basiques libres répondant à la formule I, ou on transforme un composé obtenu de formule I, dans laquelle $R^6$ désigne l'hydrogène, avec des bases organiques ou minérales, en des sels de phénolates, ou des sels obtenus correspondants sont transformés avec des acides en les composés basiques libres de formule I.

8. Composition pharmaceutique à base d'un composé suivant la revendication 1.

9. Procédé de préparation d'une composition pharmacautique suivant la revendication 8, caractérisé en ce qu'on transforme un composé suivant la revendication 1, le cas échéant avec des excipients et/ou stabilisants pharmacologiquement acceptables en une forme d'administration appropriée.

10. Composé suivant la revendication 1 pour utilisation dans le traitement de perturbations du spectre lipoprotéinique du sérum.

11. Composés de formule générale IV

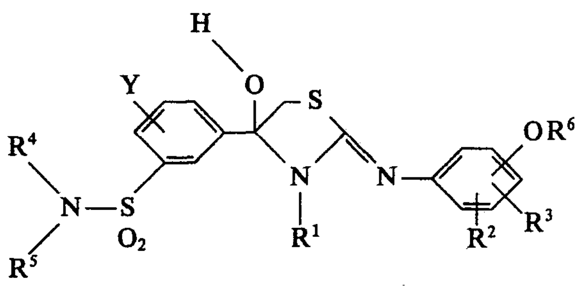

(IV)

dans laquelle $R^1$ à $R^6$ et Y ont les significations indiquées à propos de la formule I, ainsi que leurs sels.

**Revendications pour l'Etat contractant: AT**

1. Procédé de préparation de dérivés de thiazoline de formule générale I

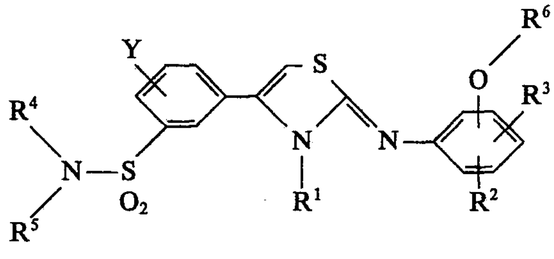

(I)

dans laquelle

$R^1$ désigne un groupe alkyle en $C_1$ à $C_3$,

$R^2$ et $R^3$ désignent l'hydrogène, un halogène, un groupe alkyle ou alcoxy avec à chaque de 1 à 4 atomes de C, $R^2$ et $R^3$ étant identiques ou différents,

$R^4$ et $R^5$ désignent l'hydrogène ou un groupe alkyle en $C_1$ à $C_4$, $R^4$ et $R^5$ étant identiques ou différents, ou forment avec l'atome de N un cycle saturé avec jusqu'à 6 chainons dans le cycle,
$R^6$ désigne l'hydrogène ou un groupe acyle en $C_1$ à $C_4$ et
Y désigne l'hydrogène, un halogène ou un groupe méthyle, caractérisé en ce que

a) on fait réagir des composés de formule générale II

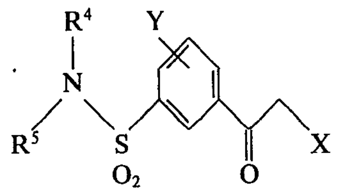

(II)

dans laquelle $R^4$, $R^5$ et Y ont la signification indiquée, X est un groupe qui part, dans des conditions de réaction condensantes, avec une thiourée de formule générale III

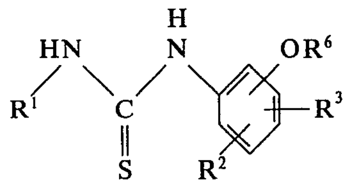

(III)

dans laquelle $R^1$, $R^2$, $R^3$, $R^6$ ont la signification indiquée, ou

b) à partir de composés de formule générale IV

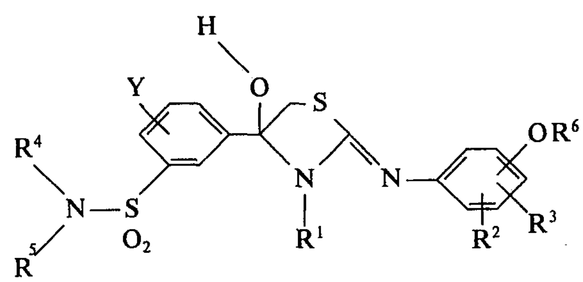

(IV)

dans laquelle $R^1$ à $R^6$ et Y ont la signification indiquée, on élimine de l'eau, ou

c) on fait réagir des composés de formule générale V

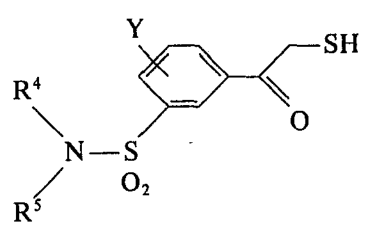

(V)

avec des composés de formule générale VI

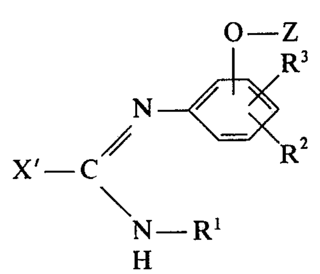

(VI)

$R^1$ à $R^5$ ayant la signification indiquée, Z désignant un groupe protecteur du groupe hydroxyle phénolique et X' étant un groupe qui part, ou

d) on transforme des composés de formule générale VII

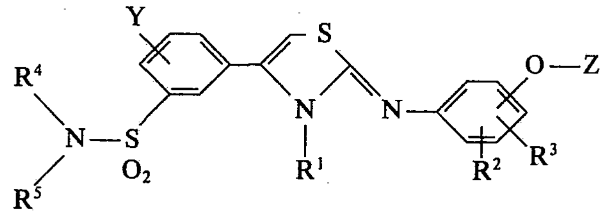

(VII)

dans laquelle $R^1$ à $R^5$ et Y ont la signification indiquée à propos de la formule I et Z a la signification indiquée à propos de la formule VI, par une scission hydrolytique ou solvolytique, en les composés de formule I, dans laquelle $R^6$ désigne l'hydrogène, ou

e) on transforme des composés de formule générale I dans laquelle $R^1$ à $R^5$ et Y ont la signification indiquée et $R^6$ représente l'hydrogène, avec un agent d'acylation en composés, de formule I avec $R^6$ désignant un acyle en $C_1$ à $C_4$, et le cas échéant un des composés de formule générale I obtenus par les modes opératoires a) à e) est transformé avec des acides organiques ouminéraux de formule générale H—A en ses sels d'addition avec des acides ou les sels des composés de formule générale I obtenus sont transformés avec des bases en les composés basique libres répondant à la formule I, ou on transforme un composé obtenu de formule I, dans laquelle $R^6$ désigne l'hydrogène, avec des bases organiques ou minérales, en des sels de phénolates, ou des sels obtenus correspondants sont transformés avec des acides en les composés basiques libres de formule I.

2. Procédé suivant la revendication 1, caractérisé en ¢e qu'on prépare des dérivés de thiazoline de formule générale I indiquée dans la revendication 1, dans lesquels:

$R^1$ désigne un groupe méthyle ou éthyle,
$R^2$ désigne l'hydrogène ou le chlore,
$R^3$ désigne l'hydrogène ou le chlore ou un groupe méthyle,
$R^4$ désigne l'hydrogène ou un groupe méthyle ou éthyle,
$R^5$ désigne l'hydrogène ou un groupe méthyle,
$R^6$ désigne l'hydrogène ou $-CO-CH_3$ ou $-COC_2H_5$,
Y désigne le chlore, le brome ou un groupe méthyle en position 4, 5 ou 6 par rapport au cycle thiazoline (le radical sulfamoyle étant fixé en position 3),

ainsi que leurs sels physiologiquement acceptables.

3. Procédé suivant la revendication 1, caractérisé en ce qu'on prépare des dérivés de thiazoline de formule générale I indiquée dans la revendication I, dans lesquels:

$R^1$ désigne un groupe méthyle,
$R^2$ et $R^3$ désignent l'hydrogène,
$R^4$ désigne l'hydrogène ou un groupe méthyle,
$R^5$ désigne l'hydrogène ou un groupe méthyle,
$R^6$ désigne l'hydrogène, le group OH étant en position 4 par rapport au groupe imino,
Y désigne un chlore en position 4 par rapport à la thiazoline, .

ainsi que leurs sels physiologiquement acceptables.

4. Procédé suivant la revendication 1, caractérisé en ce qu'on prépare la 4-(4-chloro-3-sulfamoylphényl)-2-(4-hydroxyphénylimino)-3-méthyl-4-thiazoline ou ses sels d'addition avec des acides physiologiquement acceptables.

5. Procédé suivant la revendication 1, caractérisé en ce qu'on prépare le 4-(4-chloro-3-méthylsulfamoylphényl)-2-(4-hydroxyphénylimino)-3-méthyl-4-thiazoline ou ses sels d'addition avec des acides pharmacologiquement acceptables.

6. Procédé suivant la revendication 1, caractérisé en ce qu'on prépare la 4-(4-chloro-3-diméthylsulfamodylphényl)-2-(4-hydroxyphénylimino)-3-méthyl-4-thiazoline ou ses sels d'addition avec des acides pharmacologiquement acceptables.